# EUROPEAN PATENT APPLICATION

(11) **EP 4 095 255 A1**
(43) Date of publication of application: **30.11.2022**
(21) Application number: 21744596.4
(22) Date of filing: 25.01.2021
(51) Int. Cl.: C12Q 1/68, C07K 14/485

(54) **RELATED TARGET FOR TREATING FIBROTIC DISEASES AND APPLICATIONS THEREOF**

(30) Priority: 23.01.2020 CN 202010076729; 03.11.2020 CN 202011212639
(71) Applicant: SHANGHAI SYNVIDA BIOTECHNOLOGY CO. LTD., Shanghai 201303 (CN)
(72) Inventor: LIU, Junling, Chongqing South Road, Huangpu District Shanghai 200025 (CN); FAN, Xuemei, Chongqing South Road, Huangpu District Shanghai 200025 (CN); GENG, Yan, Chongqing South Road, Huangpu District Shanghai 200025 (CN); LI, Lin, Chongqing South Road, Huangpu District Shanghai 200025 (CN)
(74) Representative: Biggi, Cristina
(86) International application number: PCT/CN2021/073547
(87) International publication number: WO 2021/148022

(57) **Abstract**

Disclosed are Pear 1 and, serving as a target of fibrotic diseases, applications in a medicament for treating human Pear 1-related diseases. Also disclosed are a Pear 1-targeting antibody or a mutant of same or a composition comprising same and applications thereof. Disclosed are a humanized Pear 1 transgenic mice model, a construction method for same, and applications thereof. The technical solution of the present invention regulates fibroblast activation and has broad application prospects in treating fibrotic diseases and accompanying debilitating diseases.

## Description

The present application claims the priority of Chinese patent application 202010076729.2 filed on January 23, 2020 and Chinese patent application 20201128639.8 filed on November 3, 2020, the contents of which are incorporated herein by reference in its entirety.

### Technical Field

The invention belongs to the field of biomedicine, and refers to a target relating treating fibrotic diseases and application thereof, in particular to application of Pear 1 as a target in treatment of fibrosis-related diseases, and refers to an antibody targeting Pear 1 for treating fibrosis-related diseases and application thereof.

### Background of the Invention

All of the organ or tissue damages will trigger complex cell and molecular reactions, ultimately resulting in fibrosis of the tissues. While such a tissue fibrosis reaction may have a feature of adaptive tissue repair within a short term, an organ or tissue scar will occur when the fibrosis is in a long term progress, eventually leading to tissue and cell dysfunction and organ failure. Due to known or unknown reasons, most human organs and tissues such as skin, lung, liver, heart, kidney, pancreas, spleen, nervous system and bone marrow can be fibrotic, which seriously harms human health. Major pathological change of fibrosis is the increase of the connective tissues and the reduced parenchymal cells in organ tissues. The continuous progress can cause damages to the structure and the function, even cause organ failure, and is a serious threat to human health and life. Tissue fibrosis is a major cause of disability and lethality of many diseases worldwidely. Nearly 45% of the death from various diseases can be ascribed to tissue fiber hyperplasia conditions. The main mechanism of the organ fibrosis is essentially chartered with fibroblast proliferation, huge amounts of accumulated extracellular matrix accompanied with inflammatory injuries and destroyed tissue structures.

However, the damages to the structure and the function of fibroblasts and extracellular matrix in the normal tissues cannot be prevented or reversed at present. It is urgent for the field to develop a medicine for improving the occurrence, progression and deterioration of fibrotic diseases thereby restoring the normal function of the tissues and the organs.

### Summary of Invention

In order to overcome the defects in the prior art that no drug has been developed for improving fibrosis disease occurrence, development and deterioration thereby restoring normal function of the tissues and the organs, the present invention provides a target, namely Pearl, for treating fibrotic disease and application thereof; in particular, provides an antibody targeting Pearl for treating fibrosis-related diseases.

A first aspect of the present invention provides an application of a gene, a protein of Pearl (Platelet Endothelial Aggregation Receptor1) and agonist thereof in 1) preparing a drug for treating fibrotic diseases; 2) preparing a drug for down-regulating and/or inhibiting fibroblast activation signaling pathway; and/or 3) preparing a drug for inhibiting fibroblast activation.

In another embodiment, the fibrotic disease includes pulmonary fibrosis, hepatic fibrosis, cardiac fibrosis, renal fibrosis, bone marrow fibrosis, skin scar, and/or soft tissue fibrosis.

In another embodiment, the pulmonary fibrosis is idiopathic pulmonary fibrosis (IPF). Preferably, the pulmonary fibrosis further comprises restrictive lung disease accompanied with pulmonary fibrosis.

In another embodiment, the cardiac fibrosis is a myocardial fibrosis, in particular myocardial fibrosis after myocardial infarction.

In another embodiment, the skin fibrosis comprises a disease in which normal tissue cells being replaced by fibroblasts due to traumatic factors in skin or soft tissues.

In another embodiment, the traumatic factors include scars formed in skin resulted from trauma, empyrosis, surguries, or plastic surguries, or cheloid or scleroderma scars formed in skin of those with scar diathesis , or, etc.

In another embodiment, the agonist is a Pearl protein agonist.

In another embodiment, the agonist enhances expression and/or activity of Pearl.

In another embodiment, the agonist comprises an antibody targeting Pearl or a variant thereof, Fc ε R1α, dextran sulfate, fucose, or a combination thereof.

In another embodiment, the agonist comprises an antibody targeting Pearl or a variant thereof.

In another embodiment, the agonist is an antibody or a variant thereof according to the second aspect of the present invention

In another embodiment, Pearl comprises a Pearl gene and/or an encoded protein thereof.

In another embodiment, Pear 1 gene or the encoded protein is derived from a mammal.

In another embodiment, Pearl gene or the encoded protein is derived from human, mouse, rat, cat, dog, monkey, camel, alpaca, or a combination thereof.

In another embodiment, Pearl gene or the encoded protein thereof is derived from human or mouse.

In another embodiment, the fibroblast activation signaling pathway comprises a PI3K/AKT signaling pathway, a MAPK signaling pathway and/or a SMAD signaling pathway.

According to the second aspect of the present invention, an isolated antibody or a variant thereof targeting Pear 1 is provided, the antibody comprises a heavy chain variable region and/or a light chain variable region; amino acid sequences of HCDR1, HCDR2 and HCDR3 of the heavy chain variable region are shown as SEQ ID NO: 26, SEQ ID NO: 27, and SEQ ID NO: 28, respectively; amino acid sequences of LCDR1, LCDR2 and LCDR3 of the light chain variable region are shown as SEQ ID NO: 45, GAT and SEQ ID NO: 46, respectively, or amino acid sequences of LCDR1, LCDR2 and LCDR3 of the light chain variable region are shown as SEQ ID NO: 47 GTS, and SEQ ID NO: 48, respectively, or amino acid sequences of LCDR1, the LCDR2 and the LCDR3 of the light chain variable region are shown as SEQ ID NO: 49, DTS, and SEQ ID NO: 50, respectively, or amino acid sequences of LCDR1, LCDR2 and LCDR3 of the light chain variable region are shown as SEQ ID NO: 51, GAT, and SEQ ID NO: 52, respctively, or amino acid sequences of LCDR1, LCDR2 and LCDR3 of the light chain variable region are shown as SEQ ID NO: 53, LVS, and SEQ ID NO: 54, respcetively.

In another embodiment, the variant is a sequence with one to several, preferably 1, 2, 3, 4, 5, 6, 7, 8, 9, or 10 amino acid residues addition, deletion or substitution of CDR, FR or full length sequence of the antibody.

In another embodiment, the variant has or substantially has an activity of targeting Pearl.

In another embodiment, HCDR1 of the variant is mutated from S to A or T at the third position, from G to D at the sixth position and/or from P to T or Y at the eighth position of amino acid sequence as shown in SEQ ID NO: 26, and/or HCDR2 of the variant is mutated from P to S or G on the third position, from Y to N at the fourth position, and/or from T to A at the eighth position of amino acid sequence as shown in SEQ ID NO: 27, and/or, HCDR3 of the variant is mutated from A to D in the ninth position, and/or from Y to F at the tenth position of the amino acid sequence as shown in SEQ ID NO: 28.

In another embodiment, HCDR1 of an antibody or a variant thereof comprises any one of amino acid sequence selected from the group consisting of SEQ ID NOs: 26, 31, 33, 36 and SEQ ID NO: 39. HCDR2 comprises any one of amino acid sequence selected from SEQ ID NOs: 27, 30, 34, 37, and SEQ ID NO: 40-44; and HCDR3 comprises any one of amino acid sequence selected from the group consisting of SEQ ID NOs: 28, 29, 32, 35 and SEQ ID NO 38; and/or,

LCDR 1 of the antibody or a variant thereof comprises any one of amino acid sequence selected from SEQ ID NOs: 45, 47, 49, 51 and SEQ ID NO: 53; LCDR2 comprises any one of amino acid sequence selected from GAT, SAS, DTS, and LVS; and LCDR3 comprises any one of amino acid sequence selected from SEQ ID NO: 46, 48, 50, 52 and SEQ ID NO: 54.

In another embodiment, HFR (heavy chain variable region framework region) 1 of the antibody or variant thereof comprises any one of amino acid sequence selected from SEQ ID NOs: 114, 115 and SEQ ID NO: 116. HFR2 comprises any one of amino acid sequence selected from SEQ ID NOs: 117, 118, 119, 120, 121, 122, 123, 124 and SEQ ID NO: 125; and HFR3 comprises any one of amino acid sequence selected from the group consisting of SEQ ID NOs: 126, 127, 128, 129, 130, 131, 132, 133, 134 and SEQ ID NO: 135; and HFR4 comprises any one of amino acid sequence selected from the group consisting of SEQ ID NO: 136 and SEQ ID NO: 137; and/or,

In another embodiment, LFR (light chain variable region framework region) 1 of the antibody or variant thereof comprises any one of amino acid sequence selected from SEQ ID NOs: 138, 139, 140, 141, 142 and SEQ ID NO: 143. LFR2 comprises any one of amino acid sequence selected from SEQ ID NO: 144, 145, 146, 147, 148 and SEQ ID NO: 149; and LFR3 comprises any one of amino acid sequence selected from SEQ ID NO: 150, 151, 152, 153, 154, 155, 156 and SEQ ID NO: 157; and LFR4 comprises any one of amino acid sequence selected from SEQ ID NO: 158, 159, 160, 161 and SEQ ID NO: 162.

In another embodiment, the antibody or variant thereof comprises an amino acid sequences as shown respectively:
HCDR1, HCDR2 and HCDR3 as shown in SEQ ID NO: 26, SEQ ID NO: 27, and SEQ ID NO: 28, LCDR1, LCDR2 and LCDR3 as shown in SEQ ID NO: 45, GAT, and SEQ ID NO: 46; or,
HCDR1, HCDR2 and HCDR3 as shown in SEQ ID NO: 26, SEQ ID NO: 27, and SEQ ID NO: 29, LCDR1, LCDR2 and LCDR3 as shown in SEQ ID NO: 45, GAT, and SEQ ID NO: 46; or,
HCDR1, HCDR2 and HCDR3 as shown in SEQ ID NO: 26, SEQ ID NO: 30, and SEQ ID NO: 28, LCDR1, LCDR2 and LCDR3 as shown in SEQ ID NO: 47, GTS, and SEQ ID NO: 48,
HCDR1, HCDR2 and HCDR3 as shown in SEQ ID NO: 26, SEQ ID NO: 27, and SEQ ID NO: 29, LCDR1, LCDR2 and LCDR3 as shown in SEQ ID NO: 49, DTS, and SEQ ID NO: 50; or,
HCDR1, HCDR2 and HCDR3 as shown in SEQ ID NO: 26, SEQ ID NO: 40, and SEQ ID NO: 28, LCDR1, LCDR2 and LCDR3 as shown in SEQ ID NO: 49, GAT, and SEQ ID NO: 54; or
HCDR1, HCDR2 and HCDR3 as shown in SEQ ID NO: 26, SEQ ID NO: 41, and SEQ ID NO: 28, LCDR1, LCDR2 and LCDR3 as shown in SEQ ID NO: 49, SAS, and SEQ ID NO: 54; or
HCDR1, HCDR2 and HCDR3 as shown in SEQ ID NO: 26, SEQ ID NO: 42, and SEQ ID NO: 28, LCDR1, LCDR2 and LCDR3 as shown in SEQ ID NO: 49, LVS, and SEQ ID NO: 52; or
HCDR1, HCDR2 and HCDR3 as shown in SEQ ID NO: 26, SEQ ID NO: 43, and SEQ ID NO: 32, LCDR1, LCDR2 and LCDR3 as shown in SEQ ID NO: 45, GAT, and SEQ ID NO: 54; or
HCDR1, HCDR2 and HCDR3 as shown in SEQ ID NO: 31, SEQ ID NO: 27, and SEQ ID NO: 32, and LCDR1, LCDR2 and LCDR3 as shown in SEQ ID NO: 51, GAT, and SEQ ID NO: 52; or,
HCDR1, HCDR2 and HCDR3 as shown in SEQ ID NO: 31, SEQ ID NO: 37, and SEQ ID NO: 32, LCDR1, LCDR2 and LCDR3 as shown in SEQ ID NO: 53, LVS, and SEQ ID NO: 46; or,
HCDR1, HCDR2 and HCDR3SEQ ID NO: 31, SEQ ID NO: 34, and SEQ ID NO: 32, and LCDR1, LCDR2 and LCDR3 as shown in SEQ ID NO: 45, SAS, and SEQ ID NO: 50,
HCDR1, HCDR2 and HCDR3 as shown in SEQ ID NO: 31, SEQ ID NO: 30 and SEQ ID NO: 35, and LCDR1, LCDR2 and LCDR3 as shown in SEQ ID NO: 47, GAT and SEQ ID NO: 50; or,
HCDR1, HCDR2 and HCDR3 as shown in SEQ ID NO: 31, SEQ ID NO: 42, and SEQ ID NO: 28, and LCDR1, LCDR2 and LCDR3 as shown in SEQ ID NO: 49, DTS, and SEQ ID NO: 54; or,
HCDR1, HCDR2 and HCDR3 as shown in SEQ ID NO: 33, SEQ ID NO: 34, and SEQ ID NO: 35, and LCDR1, LCDR2 and LCDR3 as shown in SEQ ID NO: 45, GAT, and SEQ ID NO: 46; or,
HCDR1, HCDR2 and HCDR3 as shown in SEQ ID NO: 33, SEQ ID NO: 37, and SEQ ID NO: 32, and LCDR1, LCDR2 and LCDR3 as shown in SEQ ID NO: 53, LVS, and SEQ ID NO: 46; or,
HCDR1, HCDR2 and HCDR3 as shown in SEQ ID NO: 33, SEQ ID NO: 30, and SEQ ID NO: 35, and LCDR1, LCDR2 and LCDR3 as shown in SEQ ID NO: 47, GAT, and SEQ ID NO: 50; or,
HCDR1, HCDR2 and HCDR3 as shown in SEQ ID NO: 33, SEQ ID NO: 42, and SEQ ID NO: 28, and LCDR1, LCDR2 and LCDR3 as shown in SEQ ID NO: 49, DTS, and SEQ ID NO: 54; or,
HCDR1, HCDR2 and HCDR3 as shown in SEQ ID NO: 36, SEQ ID NO: 37, and SEQ ID NO: 38, and LCDR1, LCDR2 and LCDR3 as shown in SEQ ID NO: 53, LVS, and SEQ ID NO: 54; or,
HCDR1, HCDR2 and HCDR3 as shown in SEQ ID NO: 36, SEQ ID NO: 34, and SEQ ID NO: 32, and LCDR1, LCDR2 and LCDR3 as shown in SEQ ID NO: 45, SAS, and SEQ ID NO: 50; or,
HCDR1, HCDR2 and HCDR3 as shown in SEQ ID NO: 36, SEQ ID NO: 30, and SEQ ID NO: 35, and LCDR1, LCDR2 and LCDR3 as shown in SEQ ID NO: 47, GAT, and SEQ ID NO: 50; or,
HCDR1, HCDR2 and HCDR3 as shown in SEQ ID NO: 36, SEQ ID NO: 42, and SEQ ID NO: 28, LCDR1, LCDR2 and LCDR3 as shown in SEQ ID NO: 49, DTS, and SEQ ID NO: 54; or
HCDR1, HCDR2 and HCDR3 as shown in SEQ ID NO: 39, SEQ ID NO: 27, and SEQ ID NO: 28, and LCDR1, LCDR2 and LCDR3 as shown in SEQ ID NO: 45, GAT, and SEQ ID NO: 46; or
HCDR1, HCDR2 and HCDR3 as shown in SEQ ID NO: 39, and SEQ ID NO: 32, and LCDR1, LCDR2 and LCDR3 as shown in SEQ ID NO: 45, SAS, and SEQ ID NO: 50; or,
HCDR1, HCDR2 and HCDR3 as shown in SEQ ID NO: 39, SEQ ID NO: 30, and SEQ ID NO: 35, and LCDR1, LCDR2 and LCDR3 as shown in SEQ ID NO: 47, GAT, and SEQ ID NO: 50; or,
HCDR1, HCDR2 and HCDR3 as shown in SEQ ID NO: 39, SEQ ID NO: 42, and SEQ ID NO: 28, and LCDR1, LCDR2 and LCDR3 as shown in SEQ ID NO: 49, DTS, and SEQ ID NO: 54; or
HCDR1, HCDR2 and HCDR3 as shown in SEQ ID NO: 39, SEQ ID NO: 37, and SEQ ID NO: 28, and LCDR1, LCDR2 and LCDR3 as shown in SEQ ID NO: 49, GAT, and SEQ ID NO: 48; or
HCDR1, HCDR2 and HCDR3 as shown in SEQ ID NO: 39, SEQ ID NO: 40, and SEQ ID NO: 28, and LCDR1, LCDR2 and LCDR3 as shown in SEQ ID NO: 45, GAT, and SEQ ID NO: 46; or
HCDR1, HCDR2 and HCDR3 as shown in SEQ ID NO: 39, SEQ ID NO: 41, and SEQ ID NO: 28, and LCDR1, LCDR2 and LCDR3 as shown in SEQ ID NO: 45, GAT, and SEQ ID NO: 46; or
HCDR1, HCDR2 and HCDR3 as shown in SEQ ID NO: 39, SEQ ID NO: 42, and SEQ ID NO: 28, and LCDR1, LCDR2 and LCDR3 as shown in SEQ ID NO: 45, GAT, and SEQ ID NO: 46; or
HCDR1, HCDR2 and HCDR3 as shown in SEQ ID NO: 39, SEQ ID NO: 43, and SEQ ID NO: 28, and LCDR1, LCDR2 and LCDR3 as shown in SEQ ID NO: 45, GAT, and SEQ ID NO: 46; or
HCDR1, HCDR2 and HCDR3 as shown in SEQ ID NO: 39, SEQ ID NO: 44, and SEQ ID NO: 28, and LCDR1, LCDR2 and LCDR3 as shown in SEQ ID NO: 45, GAT, and SEQ ID NO: 46.

In another embodiment, amino acid sequences of said light chain variable region and the heavy chain variable region are shown as SEQ ID NO: 16 and SEQ ID NO: 1, respectively; or, amino acid sequences of the light chain variable region and the heavy chain variable region are shown as SEQ ID NO: 16 and SEQ ID NO: 2, respectively; or amino acid sequences of the light chain variable region and the heavy chain variable region are respectively as shown in SEQ ID NO: 17 and SEQ ID NO: 3; or amino acid sequences of the light chain variable region and the heavy chain variable region are as shown in SEQ ID NO: 18 and SEQ ID NO: 2, respectively; or amino acid sequences of the light chain variable region and the heavy chain variable region are shown as SEQ ID NO: 19 and SEQ ID NO: 4, respectively; or amino acid sequences of the light chain variable region and the heavy chain variable region are as shown in SEQ ID NO: 20 and SEQ ID NO: 5 respectively; or, amino acid sequences of the light chain variable region and the heavy chain variable region are as shown in SEQ ID NO: 21 and SEQ ID NO: 6, respectively; or amino acid sequences of the light chain variable region and the heavy chain variable region are as shown in SEQ ID NO: 22 and SEQ ID NO: 7, respectively; or amino acid sequences of the light chain variable region and the heavy chain variable region are as shown in SEQ ID NO: 23 and SEQ ID NO: 7, respectively; or, amino acid sequences of the light chain variable region and the heavy chain variable region are shown as SEQ ID NO: 24 and SEQ ID NO: 7, respectively; or, amino acid sequences of the light chain variable region and the heavy chain variable region are shown as SEQ ID NO: 25 and SEQ ID NO: 7, respectively; or, amino acid sequences of the light chain variable region and the heavy chain variable region are as shown in SEQ ID NO: 22 and SEQ ID NO: 8 respectively; or amino acid sequences of the light chain variable region and the heavy chain variable region are shown in SEQ ID NO: 23 and SEQ ID NO: 8, respectively; or amino acid sequences of the light chain variable region and the heavy chain variable region are as shown in SEQ ID NO: 24 and SEQ ID NO, respectively; or amino acid sequences of the light chain variable region and the heavy chain variable region are as shown in SEQ ID NO: 25 and SEQ ID NO: 8, respectively; or, amino acid sequences of the light chain variable region and the heavy chain variable region are as shown in SEQ ID NO: 22 and SEQ ID NO: 9, respectively; or, amino acid sequences of the light chain variable region and the heavy chain variable region are as shown in SEQ ID NO: 23 and SEQ ID NO: 9, respectively; or amino acid sequences of the light chain variable region and the heavy chain variable region are as shown in SEQ ID NO: 24 and SEQ ID NO: 9, respectively; or, amino acid sequences of the light chain variable region and the heavy chain variable region are as shown in SEQ ID NO 25 and SEQ ID NO: 9, respectively; or, amino acid sequences of the light chain variable region and the heavy chain variable region are shown as SEQ ID NO: 22 and SEQ ID NO: 10, respectively; or, amino acid sequences of the light chain variable region and the heavy chain variable region are shown as SEQ ID NO: 23 and SEQ ID NO: 10, respectively; or, amino acid sequences of the light chain variable region and the heavy chain variable region are shown in SEQ ID NO: 24 and SEQ ID NO: 10 respectively; or amino acid sequences of the light chain variable region and the heavy chain variable region are shown in SEQ ID NO: 25 and SEQ ID NO: 10 respectively; or, amino acid sequences of the light chain variable region and the heavy chain variable region are shown as SEQ ID NO: 22 and SEQ ID NO: 11, respectively; or, amino acid sequences of the light chain variable region and the heavy chain variable region are shown as SEQ ID NO: 22 and SEQ ID NO: 12, respectively; or, amino acid sequences of the light chain variable region and the heavy chain variable region are shown in SEQ ID NO: 22 and SEQ ID NO: 13 respectively; or, amino acid sequences of the light chain variable region and the heavy chain variable region are as shown in SEQ ID NO: 22 and SEQ ID NO: 14, respectively; or amino acid sequences of the light chain variable region and the heavy chain variable region are as shown in SEQ ID NO: 22 and SEQ ID NO: 15, respectively.

In another embodiment, the variant has an identity of 80%, 85%, 90%, 95%, 96%, 97%, 98% or 99% with amino acid sequence of the antibody

In another embodiment, the antibody is a full-length antibody, Fab, Fab', F (ab') 2, Fv such as scFv, bispecific antibody, multispecific antibody, or a single domain antibody, or a monoclonal antibody or polyclonal antibody derived from the antibody.

In another embodiment, the antibody or variant thereof is a full-length antibody, and heavy chain constant region of the full-length antibody is selected from a heavy chain constant region of hlgG1, hlgG2, hlgG3, hlgG4 and a derivative sequence thereof.

In another embodiment, light chain constant region of the antibody is selected from a κ chain or a λ chain or a derivative sequence thereof.

In another embodiment, the derivative sequence of the heavy chain constant region comprises a sequence with one to several, preferably 1, 2, 3, 4, 5, 6, 7, 8, 9, or 10 amino acid residues addition, deletion or substitution of the heavy chain constant region of hlgG1, hlgG2, hlgG3 or hlgG4.

In another embodiment, the heavy chain constant region of the heavy chain constant region has an 80%, 85%, 90%, 95%, 96%, 97%, 98% or 99% identity with the heavy chain constant region of hlgG1, hlgG2, hlgG3 or hlgG4.

In another embodiment, the heavy chain constant region of the full-length antibody is a heavy chain constant region of hlgG3 or hlgG4 or a derivative sequence thereof.

In another embodiment, the derived sequence is an amino acid sequence having 1-2 amino acid(s) substitution of a heavy chain constant region of hlgG1, hlgG2, hlgG3 or hlgG4.

In another embodiment, the derivative sequence is hlgG4 S228P mutation.

In another embodiment, the light chain constant region is a human κ chain

According to a third aspect of the present invention, an isolated nucleic acid encoding any one of the antibody or the variant thereof according to the second aspect of the present invention is provided.

According to a fourth aspect of the present invention, a recombinant expression vector comprising the isolated nucleic acid according to the third aspect of the present invention is provided.

According to a fifth aspect of the present invention, a transformant comprising the isolated nucleic acid according to the third aspect of the present invention, or the recombinant expression vector according to the fourth aspect of the present invention is provided.

In another embodiment, the host of the transformant is prokaryotic or eukaryotic cells.

In another embodiment, the prokaryotic cell comprises E coli.

In another embodiment, the eukaryotic cell comprises yeast, such as Saccharomyces cerevisiae, Pichia pastoris, Aspergillus or Trichoderma or a combination thereof.

According to a sixth aspect of the present invention, a drug comprising the antibody or the variant thereof according to the second aspect of the present invention is provided.

In another embodiment, the drug is a pharmaceutical composition.

In another embodiment, the pharmaceutical composition further comprises a pharmaceutically acceptable carrier.

In another embodiment, the pharmaceutical composition comprises one or more Pearl agonists.

In another embodiment, the pharmaceutical composition further comprises one or more of Fc ε R1α, dextran sulfate, fucose, aptamer and small molecular compound as Pearl agonists; more preferably, Fc ε R1α, dextran sulfate or fucose.

In another embodiment, the pharmaceutical composition comprises an antibody targeting Pear 1 as described above, wherein the antibody comprises a full-length antibody, Fab, Fab', F (ab ') 2, Fv such as scFv, a bispecific antibody, a multispecific antibody, a single domain antibody or a monoclonal or polyclonal antibody prepared from the antibody as described above; preferably further comprises Fc ε R1 α , dextran sulfate, fucose, aptamer and small molecular compound; or comprises the antibody targeting Pear 1 as described above, and the antibody comprises a full-length antibody, Fab, Fab', F (ab ') 2, Fv such as scFv, a bispecific antibody, a multispecific antibody, a single domain antibody, or a monoclonal antibody or polyclonal antibody prepared from the antibody, preferably further comprises Fc ε R1 α , dextran sulfate, fucose, aptamer and small molecular compound; more preferably, comprises administering Fc ε R1α, dextran sulfate, or fucose.

According to a seventh aspect of the present invention, use of the antibody or the variant thereof according to any one of the second aspect of the present invention, or the transformant according to the fifth aspect of the present invention or the drug according to the sixth aspect of the present invention is provided in 1) preparing a drug for treating fibrotic diseases; 2) preparing a drug for down-regulating and/or inhibiting fibroblast activation signaling pathway; and/or 3) preparing a drug for inhibiting fibroblast activation.

In another embodiment, the fibrotic disease includes pulmonary fibrosis, hepatic fibrosis, cardiac fibrosis, renal fibrosis, bone marrow fibrosis, skin scar, and/or soft tissue fibrosis

In another embodiment, the pulmonary fibrosis further comprises restrictive lung disease accompanied with pulmonary fibrosis.

In another embodiment, the skin fibrosis comprises a disease in which normal tissue cells being replaced by fibroblasts due to traumatic factors in skin or soft tissues.

In another embodiment, various factors further include one or more of infection, trauma, medication, radiation, foreign matters, or immune hyperfunction.

According to an eighth aspect of the present invention, a non-therapeutic method is provided for inhibiting activation of fibroblast cells in vitro, comprising the steps of: adding a Pearl gene, an expression product thereof, or an agonist thereof to cultured fibroblasts, thereby inhibiting activation of fibroblasts.

In another embodiment, the activation of the fibroblasts comprises epithelial-mesenchymal transition of fibroblasts and replacing normal tissue cells.

In another embodiment, Pear 1 gene, the protein thereof, or the agonist thereof has a concentration of 0.001 nM -1 M.

According to a ninth aspect of the present invention, a method of administering the antibody or the variant thereof according to the second aspect of the present invention, or the drug according to the sixth aspect of the present invention to an individual or an object in need for treating fibrotic diseases is provided

In another embodiment, the invention provides a use of the antibody or the variant thereof according to any one of the second aspect, the transformant according to the fifth aspect of the invention, or the drug according to the sixth aspect of the invention in 1) treating fibrotic diseases; 2) down-regulating and/or inhibiting fibroblast activation signaling pathways; and/or 3) inhibiting fibroblast activation.

In another embodiment, the individual or the object in need includes an individual or an object that has a fibrotic disease.

In another embodiment, the individual or the object in need includes an individual or an object that suffering or suffered from pneumonia, hepatitis, nephritis, myocardial infarction, surgery, and/or trauma due to various factors.

In another embodiment, administered or applied formulation comprises inhalation formulation, local-administered formulation, parenteral-administered formulation, or a combination thereof.

In another embodiment, the inhalation formulation comprises nebulization formulation, spray formulation, nose drop, powder, or a combination thereof.

In another embodiment, the parenteral-administered formulation comprises drop, gel, emulsion, ointment, patch, film, injection, or a combination thereof.

In another embodiment, the parenteral-administered includes topical-administered, instillation, injection, or a combination thereof, such as subcutaneous injection.

According to the tenth aspect of the invention, a method is provided for constructing a humanized Pear 1 transgenic animal model, and the method comprises step of replacing animal Pearl gene with human Pear gene.

In another embodiment, the method comprises steps of : (i) selecting an exon of an animal Pear 1 gene; (ii) conducting homologous recombination at the exon of the animal Pear 1 gene, thereby replacing animal Pear 1 gene with human Pear 1 gene.

In another embodiment, the animal is a non-human mammal

In another embodiment, the animal comprises mouse, rat, feline, canine, monkey, camel, alpaca, or a combination thereof

In another embodiment, the exon of Pearl gene is one or more of exons 4-6 of mouse Pearl gene.

In another embodiment, the method comprises steps of (i) designing a target near exon 1-ATG of mouse coding region, and replacing the mouse Pearl gene in presence of homologous recombination template.

In another embodiment, adding a SV40 polyA transcription termination signal after human Pearl cDNA, thereby allowing mouse Pearl transcripts be completely replaced by human Pearl cDNA; and placing human Pearl gene after mouse Pearl gene promoter, thereby allowing human Peare1 gene be normally expressed in every tissue and organ of the mouse.

Preferably, the method further comprises (iii) a step of determining whether a humanized mouse is successfully constructed: amplifying human Pearl gene with primers, and determining a successful construction if human Pearl gene can be amplified; more preferably, the primers are as shown in SEQ ID NO: 110-113.

According to an eleventh aspect of the invention, a humanized Pearl transgenic mouse model constructed by the method is also provided.

According to a twelfth aspect of the present invention, a use of the humanized Pearl transgenic mouse model of the eleventh aspect of the present invention is provided in screening / evaluating a drug for treating diseases associated with human Pearl.

According to a thirteenth aspect of the present invention, a use of the humanized Pearl transgenic mouse model of the eleventh aspect of the present invention is provided in evaluating treatment effect of a drug targeting human Pearl in animal model.

According to a fourteenth aspect of the invention, a method is provided for screening a Pearl agonist comprising steps of (1) adding or not adding candidate molecules into a fibroblast culture system of a test group or a control group, respectively; (2) comparing fibroblast activation between the test group and the control group; and (3) confirming the candidate molecule is a Pearl agonist if the fibroblast activation in the test group is significantly lower than the control group.

In another embodiment, the fibroblast activation means ratio between amounts (A1) of epithelial-mesenchymal transition in the test group and amounts (A0) of fibroblast in the control group is at least greater than 1.2, or at least greater than 1.5, or at least greater than 2.0 to 10.0.

In another embodiment, the fibroblast activation means ratio between amounts (E1) of extracellular matrix of fibroblasts in the test group between amounts (E0) of extracellular matrix of fibroblasts in the control group is at least greater than 1.2, or at least greater than 1.5, or at least greater than 2.0 to 10.0.

According to a fifteenth aspect of the present invention, a method is provided for screening candidate drug targeting Pearl for treating fibrotic diseases, comprising steps of: (1) obtaining the humanized Pearl transgenic animal model according to the eleventh aspect of the present invention or a humanized Pearl transgenic animal model obtained by the method according to the tenth aspect of the present invention; (2) inducing fibrotic disease symptoms of the animals in step (1); (3) administering candidate molecule to the animals in step (2); and (4) observing improvement of fibrotic disease of the animal, thereby determine whether the candidate molecule is the candidate drug targeting Pearl for treating fibrotic diseases.

In another embodiment, the animal is a non-human mammal

In another embodiment, the steps of steps (2) and (3) may be interchanged.

In another embodiment, the inducing comprises one or more of drug induction, chemical induction, radiation induction.

In another embodiment, the inducing is induced by bleomycin. The invention provides an antibody targeting Pearl and (Fab) 2, Fab fragment, or FcR1 thereof, dextran sulfate, fucose, aptamer or small molecule compound.

The invention provides a pharmaceutical composition. The pharmaceutical composition comprises an antibody targeting Pearl and (Fab) 2, Fab fragment, or FcR1 thereof, dextran sulfate, fucose, aptamer or small molecule compound.

The invention provides a use of Pearl target in inhibiting fibroblast activation, treating pulmonary fibrosis, restriction lung diseases accompanied with pulmonary fibrosis and other fibrosis related diseases, promoting injury repair and/or wound healing.

The invention provides a use of the antibody targeting Pearl and (Fab) 2, Fab fragment, or FcR1 thereof, dextran sulfate, fucose, aptamer or small molecule compound in inhibiting fibroblast activation treating pulmonary fibrosis, restriction lung diseases accompanied with pulmonary fibrosis and other fibrosis related diseases, promoting injury repair and/or wound healing.

The invention provides a method of targeting Pearl in inhibiting fibroblast activation, treating pulmonary fibrosis, restriction lung diseases accompanied with pulmonary fibrosis and other fibrosis related diseases, promoting injury repair and/or wound healing. The method comprises administering and antibody targeting Pearl and (Fab) 2, Fab fragment, or FcR1 thereof, dextran sulfate, fucose, aptamer or small molecule compound to an individual in need.

In another embodiment, the administration comprises inhalation administration, pulmonary nebulization, local injection, transdermal absorption, subcutaneous injection, or local coating.

It should be understood that, within the scope of the present invention, the above technical features of the present invention and the technical features specifically described in the following (eg, embodiments) may be combined with each other to form a new or preferred technical solution. It will not be repeated herein due to the limited space of the present invention.

### Brief Description of the Figures

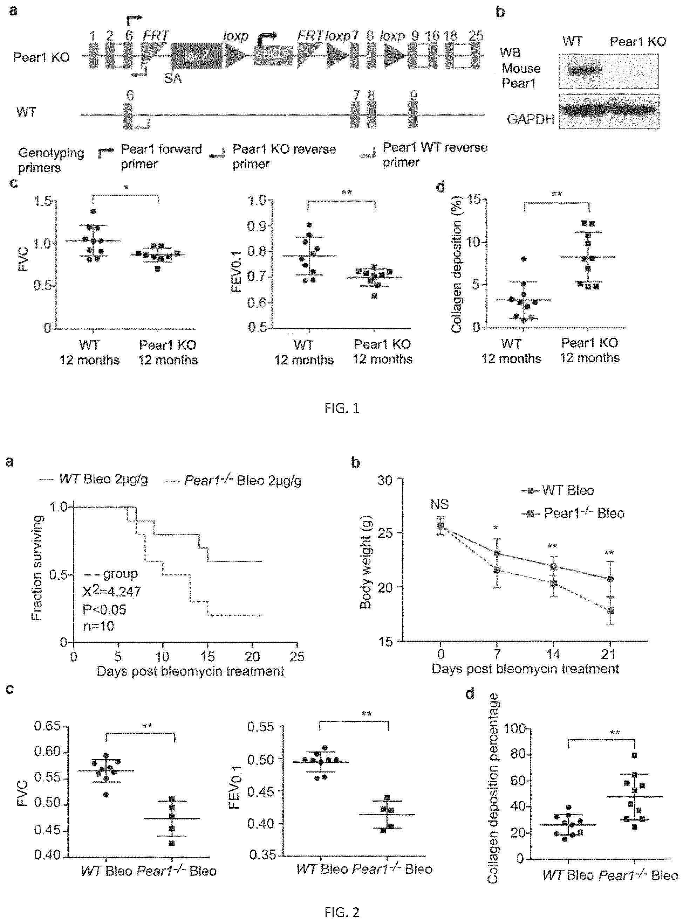

Fig. 1 Pear 1 gene deletion induces reduction of lung function of aged mice. a, the construction strategy of the Pearl knock-out mouse. b, the expression level of Pearl detected by immune-blotting experiment, and the result shows that Pearl protein is completely lost in Pearl knock-out mouse . c, Pear 1 gene deletion induces a significant decrease in the forced vital capacity (FVC) and the forced expiratory volume at 0.1 second (FEV 0.1) in the aged mice. d, Pearl gene deletion causes a significant accumulation of lung collagen components in mice.
FIG. 2 Pear 1 gene deletion aggravates the pulmonary fibrosis of the bleomycin-induced mouse. a, PEAR 1 gene deletion increased the death rate of the bleomycin-induced mice. b, PEAR 1 gene deletion increased the weight loss of the bleomycin-induced mice. c, Pearl gene deletion obviously aggravates the reduction of the forced vital capacity and the forced expiratory volume at 0.1 second (FEV 0.1) of the bleomycin-induced mouse. d, Pearl gene deletion significantly enhances the accumulation of the lung collagen components in the bleomycin induced mouse.

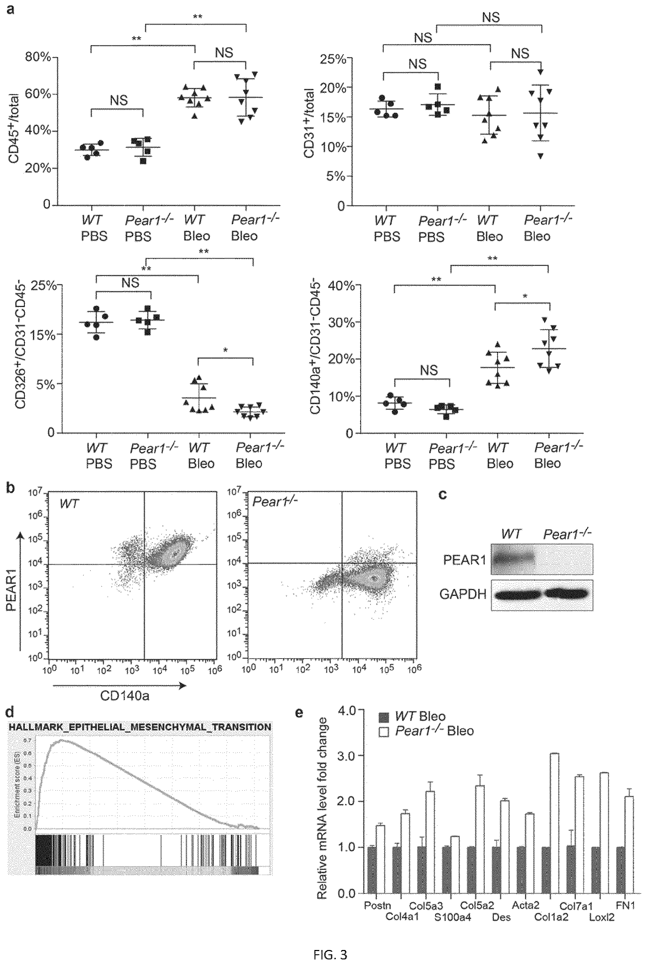

FIG. 3 Pear 1 regulates fibroblast activation and extracellular matrix synthesis through fibroblast Epithelial-Mesenchymal Transition (EMT). a, Pearl deletion has an obvious effect on the increase of the lung fibroblasts (CD140 a+) proportion and the decrease of Epithelial cells (CD326+) proportion in bleomycin-induced mice, but has little effect on CD45+ cells and endothelial cells (CD31+). b, Pearl is significantly expressed on the mouse lung fibroblasts cultured *in vitro..* c, immune-blotting confirms that Pearl is expressed on lung fibroblasts. d, Gene expression on the lung fibroblasts in the bleomycin-induced mouse by RNA-SEQ analysis; and Pearl deletion obviously promotes the epithelial-mesenchymal transition of the lung fibroblasts in the bleomycin-induced mouse. e, qPCR further confirms that the Pearl deletion obviously promotes the extracellular matrix proteins synthesized by the lung fibroblasts in the bleomycin-induced mouse.

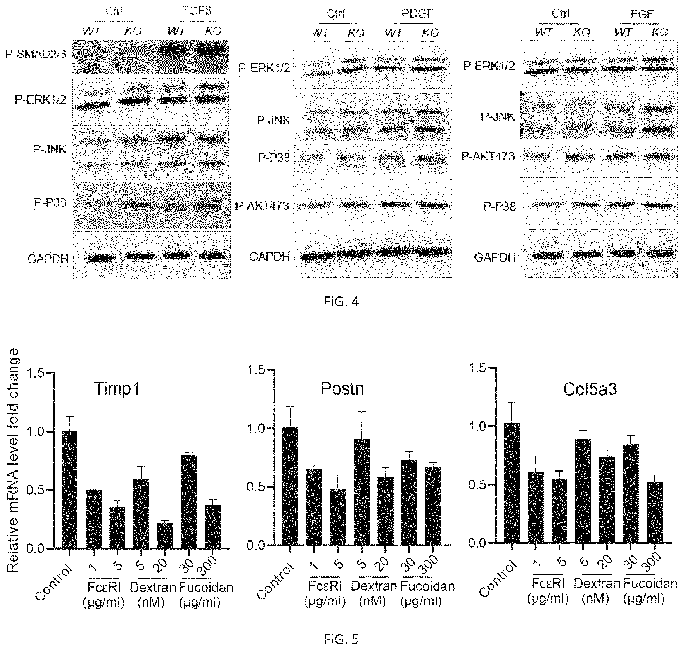

FIG. 4 Pearl deletion can significantly promote the intracellular signals mediated by transforming growth factor β (TGFβ) receptors, platelet-derived growth factor (PDGF) receptors, and fibroblast growth factor (FGF) receptors. Pearl deletion can significantly promote TGFβ receptor-mediated SMAD2/3 phosphorylation and ERK1/2, JNK, P38 phosphorylation; promote PDGF receptor-mediated ERK1/2, JNK, P38 phosphorylation and Akt phosphorylation; promote FGF receptor-mediated ERK1/2, JNK, P 38 phosphorylation and Akt phosphorylation.
FIG. 5 Activated Pearl inhibits the extracellular matrix proteins expressed by fibroblasts. The Fc ε R1α, dextran sulfate and fucose are capable of inhibiting the expressions of the extracellular matrix gene Periostin (Postn), Col5a3 and Timp1 of human lung fibroblasts.

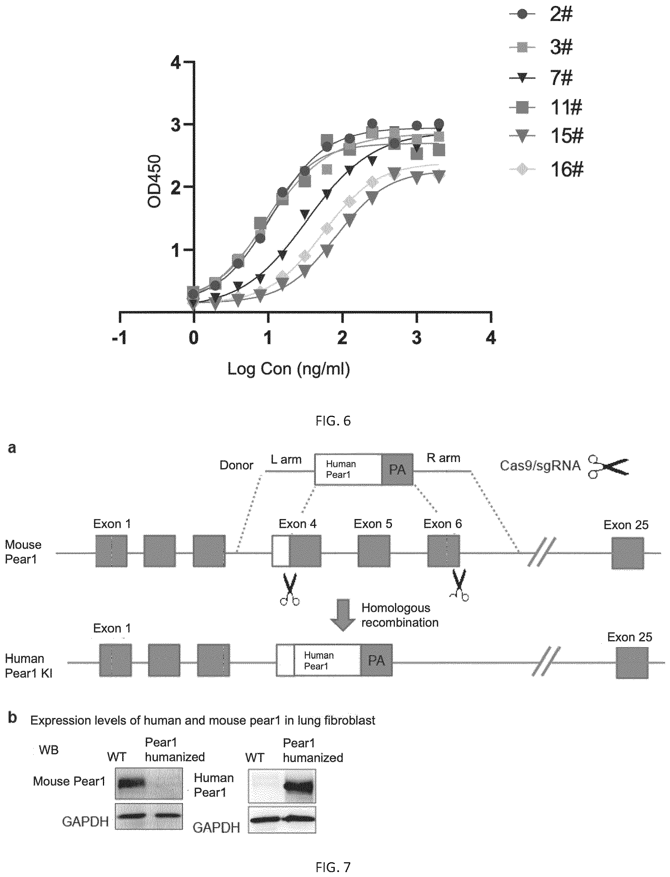

FIG. 6 The comparison of the affinities of the anti-human Pearl antibodies to human Pearl. The ELISA plate was coated with human Pearl ECD proteins and the reactions were conducted between antibodies at different concentrations (2000 ng/mL, 1000 ng/mL, 500 ng/mL, 250 ng/mL, 125 ng/mL, 62.5 ng/mL, 31.25 ng/mL, 15.625 ng/mL, 7.8125 ng/mL, 3.90625 ng/mL, 1.953125 ng/mL, 0.9765625 ng/mL) and the human Pearl ECD proteins. OD value was measured to obtain the EC 50 value of the antigen-antibody combination
FIG. 7 The anti-human Pearl antibody obviously improves the lung fibrosis process of the human Pearl transgenic mouse induced by bleomycin. a, The humanized transgenic mouse construction strategy. b, The mouse lung fibroblasts lysis was prepared and tested for the expression levels of mouse Pearl and human Pearl by immune-blotting. The result shows humanized transgenic mouse was successfully constructed.

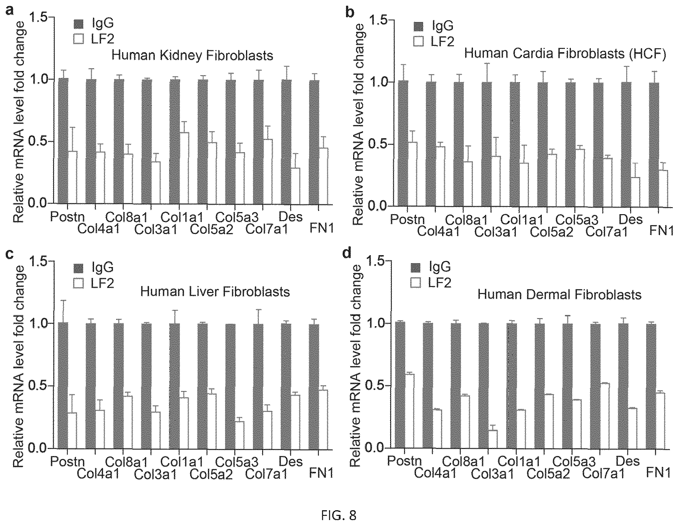

FIG. 8 The anti-human Pearl monoclonal antibody inhibits the expressions of extracellular matrix proteins in human fibroblasts derived from various tissues. a. LF2 monoclonal antibody inhibits human kidney fibroblasts to express extracellular matrix proteins. b. LF2 monoclonal antibody inhibits human cardiac fibroblasts to express extracellular matrix proteins. c. LF2 monoclonal antibody inhibits human liver fibroblasts to express extracellular matrix proteins. d. LF2 monoclonal antibody inhibits human skin fibroblasts to express extracellular matrix proteins.

### Detailed Descriptions

Through extensive and intensive research, the inventors unexpectedly found that Pearl gene and protein thereof are expressed in fibroblasts, and plays a key role in fibrotic diseases. Specifically, compared with the wild type, Pearl gene knock-out mouse constructed by the inventor significantly aggravated pulmonary fibrosis symptoms and lung function reduction upon bleomycin-induction. In addition, the inventors have also demonstrated in pathology that Pearl gene deletion can greatly promote epithelial-mesenchymal transition of fibroblasts, promote extracellular matrix synthesis capability of fibroblasts, thereby causing normal organs and tissues to present an excessive connective tissue state and causing organ fibrosis. More importantly, the inventors have demonstrated Pearl activation can reduce extracellular matrix synthesis of fibroblasts, reduce fibrosis symptoms, and even reverse fibrosis.

The inventors have also studied the effects of Pearl gene in a fibrotic disease-related signaling pathway. The experiments prove that Pearl gene deletion can up-regulate the fibrosis-related signaling pathway reported in prior arts, including one or more of up-regulation of PI3K/AKT signaling pathway, MAPK signaling pathway, and SMAD signaling pathway. Therefore, Pearl gene deletion results in up-regulation of the fibrosis-related signaling pathways and plays a key role in the balances of the over-activation of the fibroblasts, the release of the extracellular matrix, and the cell matrix generation/degradation.

Based on the above study, the inventors have constructed antibodies targeting Pearl as agonist. Antibodies of the present invention, or variants thereof, have been proved to have a pronounced function of Pearl activation, fibrosis disease-related pathways modulation (in particular down-regulating one or more of PI3K/Akt signaling pathway, MAPK signal pathway, and SMAD signaling pathway), and an excellent anti-fibrosis effect, while also demonstrating the importance of Pearl in fibrosis diseases from another perspective.

On this basis, the present invention is completed.

### Terms

As used herein, unless otherwise specified, the singular forms "a," "an, " and "the" include plural form of the referents.

As used herein, the term "substantially" is used herein to mean inherent uncertainty that may be attributed to any quantitative comparisons, values, measurements, or in other forms. The term "substantially" is also used herein to refer a quantification or a degree of referring change, which will not cause the variation of the basic function of the discussed subject matter. For example, "substantially has the activity targeting to Pearl activation" means that the antibodies are able to react with Pearl, eg, combine with Pearl protein and inhibit fibroblast activation signaling pathways to different degrees. According to the teaching of the co-relation between Pearl and fibroblasts activation, a person skilled in the art would be able to obtain the agonist of Pearl by means of conventional technics and by using screening model of the present invention.

As used herein, the term "antibody or variant thereof" refers to a protein, an antibody fragment, or a polypeptide sequence which derives from an immunoglobulin molecule that specifically binds to an antigen. The antibody may be polyclonal or monoclonal, multi-stranded or single-stranded, or an intact immunoglobulin, and may be derived from natural sources or recombinant sources. In one embodiment, the "antibody or variant" of the present invention is isolated and is derived from recombinant source.

As used herein, the term "antibody fragment" refers to at least a portion of an antibody that retains the ability to specifically interact with an antigen (eg, by binding, steric hindrance, stabilization/destabilization, spatial distribution). Examples of antibody fragments include, but are not limited to, Fab, Fab', F(ab')2, Fv fragment, scFv antibody fragment, disulfide bond-linked FVS (sdFV), Fd fragment consisting of VH and CH1 domains, linear antibodies, single domain antibodies, multi-specific antibodies formed by antibody fragments (eg, bivalent fragments of two Fab fragments linked by disulfide bonds in a hinge region) and isolated CDRs or other epitope binding fragments. The antigen-binding fragment can also be incorporated into a single-domain antibody, a maximum antibody, a micro antibody, a nano-antibody, an intracellular antibody, a bi-specific antibody, and a chimeric antibody.

As used herein, the term "derivative sequence" refers to a new sequence after one or more (eg, no more than 10) amino acid(s) addition, deletion, or conservative substitution of an original antibody/protein/polypeptide sequence; and/or a new sequence after modification of the original antibody/protein/polypeptide sequence without altering or substantially changing the activity of the original antibody/protein/polypeptide. Conservative substitutions are generally well known to those skilled in the art, for example:

| Original amino acid | Prefered conservative substitution | More preferred conservative substitutions |
|---|---|---|
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg; | Arg |
| Ile (I) | Leu; Val; Met; Ala; Phe; | Leu |
| Leu (L) | Ile; Val; Met; Ala; Phe; | Ile |
| Lys (K) | Arg; Gln; Asn; | Arg |
| Met (M) | Leu; Phe; Ile; | Leu |
| Phe (F) | Leu; Val; Ile; Ala; Tyr; | Leu |
| Pro (P) | Ala | Ala |
| Ser | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; | Leu |

As used herein, the term "nucleic acid" or "polynucleotide" refers to a single-stranded or double-stranded form of deoxyribonucleic acid (DNA) or ribonucleic acid (RNA) and a polymer thereof. Unless otherwise specified, a specific nucleic acid sequence also implies a variant thereof (eg, degenerate codon substitution), an allele, a straight homolog, an SNP and a complementary sequence, and a well-specified sequence.

As used herein, the term "expression vector" or "recombinant expression vector" can be used interchangeably, referring to a vector comprising a recombinant polynucleotide, which comprises an expression control sequence operatively linked to a nucleotide sequence to be expressed. The expression vector contains sufficient cis-acting elements for expression; other elements for expression may be provided by the host cell or in an *in vitro* expression system. Expression vectors include all of those known in the art, including those incorporated into recombinant cosmids, plasmids (eg, bare or contained in liposomes) and viruses (eg, lentiviruses, retroviruses, adenoviruses, and adeno-associated viruses).

As used herein, the term "signaling pathway" or "signal transduction pathway" can be used interchangeably, referring to a biochemical relationship between a plurality of signal transduction molecules that transduce a signal from one portion of a cell to another portion of a cell to be functional. The term "cell surface receptor" includes molecules and molecular complexes capable of receiving signals and transferring signals across a cell membrane.

As used herein, the term "specifically bind" refers to an antibody or ligand that recognizes and binds to a binding partner (eg, Pearl) protein present in a sample, but the antibody or ligand substantially does not identify or bind other molecules in the sample.

As used herein, the term "subject" or "object" can be used interchangeably referring to a living organism (eg, a mammal, such as a mouse, rat, feline, canine, sheep, camel, monkey, alpaca or human) in which an immune response can be induced.

As used herein, the terms "effective amount", "safe effective amount", or "therapeutically effective amount" can be used interchangeably herein referring to the amount of a substance, formulation, drug, or pharmaceutical composition that effectively implements a particular biological result and has an acceptable adverse reaction for the subject, as described herein. For example, a person skilled in the art, such as an experienced clinician, may adjust the "safe effective amount" according to the subject.

As used herein, the term "targeted activation" ("targeting activation") refers to a molecule to be tested having an activation effect on a gene, protein, or activity of a target, thereby up-regulating its expression or activity. Generally, "targeted activation" can be determined by one or more of methods such as detecting expression quantities, activity levels, downstream influence pathway or secretion of the gene or protein thereof.

As used herein, the term "treatment" ("treating" or "treat") refers to alleviating or improving the progression, severity, and/or duration of the fibrotic disease or improve one or more symptoms (preferably, one or more discernable symptoms) of a fibrotic disease by administering one or more therapies (eg, one or more therapeutic agents such as agonists or antibodies or variants thereof) .

Scope: In the entire disclosure, various aspects of the invention may be present in the form of ranges. It should be understood that the description in the form of ranges is for convenience and brevity and should not be construed as limiting the scope of the invention. Accordingly, the description of the ranges should be considered to specifically disclose all possible sub-ranges and individual numerical values within that range. For example, a description of a range, such as from 1 to 6, should be considered to specifically disclose sub-ranges such as 1 to 3, 1 to 4, 1 to 5, 2 to 4, 2 to 6, 3 to 6, etc. as well as individual numerical values within that range, such as 1, 2, 2.7,3,4, 5, 5.3, and 6. As another example, a range, such as having an identity of 95-99%, includes a range of identity of 95%, 96%, 97%, 98% or 99% including sub-ranges such as an identity of 96-99%, 96-98%, 96-97%, 97-99%, 97-98% and 98-99%. This is applicable regardless of the width of the range.

### Fibrotic disease

As used herein, the term "fibrotic disease" refers to disease that results from over or abnormal activation of fibroblasts due to damages to normal tissue or cell, tissue or organ repair initiated by fibroblasts, resulted epithelial-mesenchymal transition of fibroblasts, increased extracellular matrix synthesis, scar tissue substitutions of normal tissues and function loss of normal tissues.

Generally, since fibroblasts are widely distributed in various normal tissues, as long as normal tissue cells are damaged, excessive or abnormal activation of fibroblasts can be induced; such as significant pulmonary fibrosis, hepatic fibrosis or renal fibrosis after lung, liver or kidney inflammation; or, such as skin, muscle or connective tissue proliferative scars caused by surgery, trauma or the like; or, myocardial fibrosis after myocardial infarction and myelofibrosis associated with myeloproliferative diseases, etc.. It is indicated that a "fibrotic disease" is a "scar" which spreads over the organs or tissues of the whole body and is caused by over or abnormal activation of fibroblasts due to damaged normal tissues, and the scar directly limits the original normal functions of organs and tissues.

From the mechanism perspective, the fibrotic disease is characterized by fibroblasts (FB) migration to the wound and/or proliferation and massive extracellular matrix aggregation accompanied by inflammation injuries and tissue structure damages. Therefore, FBs play a very important role in the course of fibrosis diseases. FBs are the most leading cell components in connective tissues, and are differentiated from mesenchymal cells in an embryo period. Fibroblast can also exist as a form of fibrocyte, which is in its mature and quiescence status. Fibroblasts and fibrocytes can be converted to each other under certain conditions. In addition, it is reported that FBs can be turned from epithelial cells, macrophages, blood mononuclear cells, endothelial cells and other normal cells through epithelial-mesenchymal transition (EMT), and these transdifferentiation processes may be one of the important generation modes of FBs in fibrotic diseases.

Under physiological conditions, FBs synthesize and release extracellular matrix (ECM) components to form tissues, maintain organ morphology and physiological functions, and get employed in repairs after tissue damages. Under pathological conditions, FBs can be transformed into activated fibroblasts by abnormal proliferations, and synthesize and secrete massive ECM proteins; thereby inducing excessive connective tissues and organ fibrosis. In addition, ECM level is further regulated and controlled by the balance between its decomposition enzyme, namely matrix metalloproteinase (MMP) and matrix metalloproteinase tissue inhibitor (TIMPS). In the physiological condition, the synthesis and decomposition of the ECMs in the lung are in a balance; while during the pulmonary fibrosis process, the balance between MMPs/TIMPs system is destroyed, and ECMs is over-accumulated to form a scar structure, which is a key factor in the development of fibrosis. It indicates that FBs, as well as the balance of the MMPs/TIMPS play an important role in fibrotic diseases.

### Pulmonary Fibrosis

Pulmonary fibrosis is the most common and the most serious fibrosis disease, and refers to pulmonary diseases caused by abnormal activation of fibroblasts and scar formation caused by abnormal repair of alveolar tissue damage. The pulmonary fibrosis, which is mainly characterized by dry cough and progressive dyspnea, seriously deteriorates the respiratory function of the human body. The lung injury aggravates with the development of the disease and the respiratory function of the patient keeps decreasing. The main causes of death comprise the acute exacerbation of the respiratory function and the associated complications thereof

The cause of lung fibrosis in most patients is unknown, and therefore it is referred as idiopathic interstitial pneumonia. The type of disease characterized as pulmonary fibrosis among idiopathic interstitial pneumonia is referred as idiopathic pulmonary fibrosis (IPF). The continuous diffusive alveolar inflammation and alveolar structure disorder of IPF lead to pulmonary interstitial fibrosis to form permanent scars, and the lung function declines irreversibly. Finally, it leads to respiratory failure and causes death of patients. IPF usually occurs in middle-aged or elder people, in particular male groups of 50 to 70. Statistics report that the prevalence in the whole population every year is about (2-29)/10 million, and the growth trend is increasing by approximately 11% year-by-year. The cause of IPF is not clear, and is currently considered to be related to environmental factors or the use of chemotherapeutic drugs. The acute attack may also be caused by repeated infections caused by viruses, bacteria, fungi, parasites and the like. In addition, autoimmune diseases such as lupus erythematosus and other autoimmune diseases are one of the causes of the disease. The prognosis of IPF is poor, the median survival time of a patient is 2-3 years, the 5-year survival rate is lower than 30%, and therefore, it is referred as a "tumor-like disease".

Since IPF progresses unpredictably, it is quite tough to treat. The treatments to IPF mainly comprise non-drug therapy, including smoking cessation, oxygen therapy, mechanical ventilation, palliative treatment and lung rehabilitation training, etc.; and drug therapy, including hormone therapy, acid-suppression therapy, N-acetylcysteine treatment and the like. Although glucocorticoids have been used for treating pulmonary fibrosis over 50 years, the latest research proves that they are only effective in 20% of patients, and long-term use of glucocorticoids may cause severe adverse reactions. In addition, Meta-analysis of research results showed that single use of N-acetylcysteine treatment cannot reduce the patient's disease death rate, or improve the change value of forced vital capacity (FVC) or the life quality. In recent years, FDA sequentially approved anti-tumor medicine pirfenidone and nintedanib as orphan drugs to treat IPF, wherein pirfenidone is a multi-effects pyridine small molecule compound with characteristics of antiinflammatory, anti-fibrosis and anti-oxidation. The pirfenidone can significantly decrease the descending rate of forced exhalation vital capacity. After continuous administration of pirfenidone for 52 weeks, the decrease of the lung function index of the patient can be slowed down, the Progression-Free Survival (PFS) of the patient is prolonged, and meanwhile, the risk of death of the patient is reduced, but the side effects comprise photosensitivity, weakness, rash, stomach discomfort and anorexia. Nintedanib is a multi-targets tyrosine kinase small molecule inhibitor, and nintedanib can significantly reduce the absolute value of FVC drop of IPF patient and relieve the disease process to a certain extent. Although both nintedanib and the pirfenidone can reduce the deterioration speed of the lung function and improve the life quality of patients, they are only suitable for delaying the descending speed of the lung function of patients, but not improving the symptoms of the respiratory system. Thus they are only applicable to the IPF patients with mild to moderate pulmonary dysfunction, and cannot be used in severe IPF patients. In addition, pirfenidone cannot be used in patients with renal dysfunction, and nintedanib cannot be used in patients with liver dysfunction, which further limited the application of these two drugs.

### Other Organ Fibrosis

Hepatic fibrosis is a pathological process caused by abnormal repair of liver injury. Abnormal proliferation of stellate cells in connective tissues (i.e., liver fibroblasts) and a large amount of secreted extracellular matrix caused by liver injuries are the main causes. Long-term hepatic fibrosis can develop into liver cirrhosis, portal vein high pressure, liver cancer and the like.

Myocardial fibrosis refers to scars formed by abnormal proliferation of myocardial fibroblasts and huge amounts of extracellular matrix induced by myocardial cell necrosis. Myocardial fibrosis will results in heart stiffness, heart dilation, and induce arrhythmia, heart failure and the like.

Renal fibrosis refers to scars formed by abnormal deposition of kidney connective tissue fibroblast extracellular matrix (ECM) due to damages of kidney cells caused by trauma, infection, blood circulation disorder, immune-inflammatory response and etc., and the resulting renal parenchyma sclerosis and function loss.

Bone marrow fibrosis is a disease caused by abnormal deposition of extracellular matrix (ECM) of bone marrow fiber tissues, and is a type of myeloproliferative disease seriously affecting hematopoietic function

Skin or soft tissue scar formation is caused by huge amounts of ECM produced and deposited by fibroblasts migrated from skin connective tissues to wounded sites of skin or soft tissues. Abnormal scar formation may hinder the healing of chronic wounds and cause fibrosis. Generally, all causes of skin or soft tissue damage, such as surgeries, trauma, empyrosis, local or diffusive inflammation (i.e. autoimmune diseases, scleroderma, etc.) can result in abnormal scars, or excessive connective tissues of soft tissues.

### Pearl Gene or Proteins thereof

Pearl, Platelet Endothelial Aggregation Receptor 1 (Gene ID: 375033), is a 150 kDa type I transmembrane protein, and is a member of a Multiple Epidermal Growth Factor-Like domain Receptor family (MEGF) Protein family. Human Pearl contains 1037 amino acids (AA), wherein a 20 AA signal sequence, a 735 AA extracellular domain (ECD), a 21 AA transmembrane region and a 261 AA cytoplasmic region are contained. Pearls express in all mammals and have high similarity. For example, the similarity between human Pearl and mouse Pearl (Gene ID II: 73182) is 84%.

Pearl was firstly found to be expressed in vascular endothelial cells and platelets. When a platelet directly contacts with another platelet, Pearl receptors on platelet surface combine with potential ligand on contacted platelet surface in the other phase through its EMI domain, thereby resulting in intracellular signal and platelet activation. However, in recent years, literatures reported that Pearl single nucleotide polymorphism may not be directly linked with human platelet activity. Except that Pearl is mainly related to the platelet activation process, it is reported that Pearl has the function of regulating neovascularization and clearing dead neurons. Therefore, it can be seen from the above researches that Pearl may exert completely different biological functions in different tissues and organs.

While the inventors firstly found that in addition to the expression in vascular endothelial cells as confirmed at present, Pearl is also expressed in fibroblasts, and has a negative regulation effect on activation of fibroblasts.

By knocking out Pearl gene in mouse and constructing a humanized Pearl gene transgenic mouse and the like, it is clear that the deletion of Pearl gene can up-regulate one or more of the reported fibrosis-related signal pathways (including upregulation of one or more pathways including PI3K/AKT signaling pathway, MAPK signaling pathway, and SMAD signaling pathway), resulting in the upregulation of fibroblast activation signaling pathway. It can be seen that Pearl gene plays a key role in the activation of fibroblasts, the release of cell matrix, and the balance of cell matrix production/ degradation.

Therefore, by using Pearl as a target, an antibody capable of enhancing the activity of Pearl is constructed, so that over-activation of fibroblasts is inhibited, and thereby achieving the purpose of treating fibrotic diseases.

Fiber Cell Activation Signal Pathway

Fibroblasts are known to be regulated by intracellular signals mediated by a variety of cytokines via their receptors. These signal pathways of these activated fibroblasts mainly include transformed growth factor beta (TGFβ) receptor-mediated SMAD signaling pathway and MAPK signaling pathway (including ERK1/2, JNK, P38 signal pathway); platelet-derived growth factor (PDGF) receptor-mediated MAPK signaling pathway (including ERK1/2, JNK, P38 signaling pathway) and PI3K/AKT signaling pathway; fibroblast growth factor (FGF) receptor-mediated MAPK signaling pathway (including ERK1/2, JNK, P38 signaling pathway) and PI3K/AKT signaling pathway, etc.

These pathways are cellular signaling pathways which widely present in vivo, and plays an important role in proliferation and differentiation of cells. For example, TGF-β signaling pathway plays an essential role in early embryonic development and tissue organ formation, immunization, tissue repair and balance of homeostasis. In another example, MAPK signaling pathways mainly comprise four downstream signal molecules: ERK1/2, JNKS, P38S and ERK5. ERK1/2, JNKS, P38S and ERK5 are closely related in the processes of immune & inflammation, cell differentiation, apoptosis, proliferation and the like. In addition, PI3K/AKT signaling pathway can regulate cell proliferation, differentiation and apoptosis, and plays a crucial role in cell migration, adhesion, angiogenesis and extracellular matrix synthesis and secretion. The existing medicines or most in research have an effect by inhibiting activation of the signaling pathways, such as Nintedanib, approved for pulmonary fibrosis treatment, can both inhibit vascular endothelial growth factor receptor (VEGFR), said MAPK signaling pathway and PI3K/Akt signaling pathway mediated by platelet-derived growth factor receptor (PDGFR) and signaling pathwayfibroblast growth factor receptor (FGFR),signaling pathway and also causes serious systemic side effects.

The inventors prove through the experiments that Pearl gene is directly related to the above signaling pathways, which is different from the effect of directly inhibiting or down-regulating the pathway activation of the reported drugs. The inventors define the negative regulation effects of Pearl gene or protein thereof on said signaling pathways, that is, the activation of these signal pathways can be promoted by knocking out Pearl, and Pearl agonist can partially down-regulate the activation of these signal pathways, thereby regulating abnormal activation of fibroblasts treat or improve fibrosis diseases.

### Agonist and Drugs

As used herein, the terms "agonist", "Pearl agonist" or "agonist of Pearl gene or a protein thereof" may be used interchangeably to refer to substances capable of enhancing the activity of Pearl gene or protein thereof, and/or binding Pearl protein, and/or promoting Pearl to inhibit signaling pathways of fibroblast activationsignaling pathway, and/or reducing ECM released from fibroblasts. In addition to the known Pearl agonist, the present invention provides a novel antibody that specifically binds to Pearl.

Preferably, the agonist provided by the present invention is an agonist which is capable of binding to a Pearl gene or a protein thereof and resulting a structure change of Pearl, thereby inhibiting fibroblast activation. Preferably, said agonist is an agonist that targets and binds to Pearl protein expressed on fibroblasts.

In a preferred embodiment, in addition to the antibody of the present invention, the agonist of the present invention may further comprise a substance known to art that has a function of activating Pearl, such as Fc ε R1α (ie, Fc fragment of IgE receptor la), dextran sulfate, fucose, or a combination thereof, and may also include natural aptamer to Pearl and/or small molecule compound, more preferably, Fc ε R1α, dextran sulfate and fucose, which can significantly inhibit ECM synthesized by lung fibroblasts, improve lung function and increase survival rate.

By using Pearl as a target of the present invention, a person skilled in the art would be able to design a variety of different agonists targeting Pearl, including, but not limited to, small molecule agonists, antibodies or active fragments thereof, miRNAs, and the like. In the present invention, the small molecule compound may also refer to a naturally occurred or artificially developed small molecule agonist to Pearl receptor.

In the present invention, the aptamer refers to an in vitro screening technology: systematic evolution of ligands by exponential enrichment (SELEX). The obtained structured oligonucleotide sequence (RNA or DNA) has high recognition capability and high affinity with corresponding target molecules (proteins, viruses, bacteria, cells, heavy metal ions and the like).

The invention also provides a drug comprising a safe and effective amount of the antibody of the invention or a variant thereof. The drug of the present invention may also be a pharmaceutical composition comprising one or more Pearl agonists. In another embodiment, the pharmaceutical composition comprises an antibody of the present invention targeting Pearl or a variant thereof, such as an antibody (Fab) 2 and a Fab fragment, Fc ε R1 α , dextran sulfate, fucose, aptamer or small molecule compound.

In another embodiment, the drug is a pharmaceutical composition, i.e. further comprises a pharmaceutically acceptable carrier or excipient. The pharmaceutically acceptable carrier of the present invention can be used to maintain the structure and function of the agonist of the present invention and easy to use. A skilled in the art can select and combine according to needs. Such carriers include, but are not limited to, saline, buffer, glucose, water, glycerol, ethanol, and combinations thereof.

Agonists, antibodies, and variants thereof, and drugs and/or pharmaceutical compositions containing them can be administered (delivered) to subjects in need by a variety of routes including, but not limited to, inhalation, topical administration, and/or parenteral administration

In a preferred embodiment, a skilled in the art may determine the type of formulation to be administered according to the route of administration, for example, including inhalation administration, local administration, parenteral administration, or a combination thereof. The inhalation administration formulation includes nebulization formulation, spray formulation, nose drop, powder, or a combination thereof. The local administration formulation and/or parenteral administration formulation includes drop, gel, emulsion, ointment, patch, film, injection, or a combination thereof. In a preferred embodiment, the drug may be administered via various routes, such as local coating, instillation, intravascular injection, local injection, etc..

### Antibody or Variant thereof

The present invention provides an antibody or a variant thereof that specifically binds to/targets a Pearl protein. Preferably, the antibodies or variants thereof of the invention are monoclonal antibodies derived from mice, and could be recombinant, polyclonal, multi-chained or single-chained, or intact immunoglobulin. Preferably, the antibodies of the invention or variants thereof are humanized. Preferably, the antibodies of the invention are chimeric antibodies. Preferably, the antibodies of the invention or variants thereof are shown in Tables 3.2, 3.3, 4 and 5.

The antibody targeting Pearl comprises a full-length antibody and an antibody fragment (such as Fab', Fab', F(ab')₂, Fv such as scFv), and also comprise a bispecific antibody, a multispecific antibody, a single domain antibody or a monoclonal antibody or polyclonal antibody prepared from the antibody.

In the present invention, the Fab fragment of the antibody is also called a fragment of antigen binding, and refers to the domain in an antibody structure that binds to an antigen. Fab fragment consists of a complete light chain and VH and CH1 domain of a heavy chain. Under the action of papain, the antibody can be degraded into two Fab fragments and an Fc fragment.

FR fragments of Fab which can be used in the antibody of the invention or the variant thereof is not particularly limited, preferably, FR of human IgG4 and many mutated FR sequences are used in the present invention. The result shows that changing in FR sequence barely affects the binding between the antigen and the antibody. It could be seen that the CDR fragments of the antibody of the present invention has an excellent ability to specifically and stably bind to PEAR 1 protein. A person skilled in the art would have been able to use the antibodies of the present invention or variants thereof according to the teaching of Pearl as a target of the present invention and the relevance to fibrotic diseases thereof, and use a plurality of currently known FR to screen the constructed antibodies, thereby obtaining an antibody having consistent or substantially consistent activity with the antibodies of the present invention or the variants thereof, and it is also included in the scope of the present invention.

Constant regions that can be used in the antibodies or variants thereof of the present invention are not particularly limited. A person skilled in the art would have been able to screen a constant region fragment suitable for an antibody containing the antibody or a variant thereof according to the teaching of Pearl as a target of the present invention and the relevance to fibrotic diseases thereof, thereby obtaining a complete antibody having consistent or substantially consistent activity with the antibodies or variants of the present invention.

### Treating Method

The invention provides a method for treating fibrosis diseases. The method comprises administering to an individual or subject in need the antibody of the invention the variant thereof or the drug of the invention. In another embodiment, the treating method is a method for treating pulmonary fibrosis, restriction lung diseases accompanied with pulmonary fibrosis. The method comprises, but not limited to, administering the antibodies of the invention or the variants thereof or the drug of the invention via nebulization.

In another embodiment, the treatment method is a method for treating skin or soft tissue fibrosis. The method comprises, but is not limited to, administering the antibodies of the invention or the variants thereof via the route of, such as local injection, external application, coating, infiltration, transdermal application, or the like. Preferably, for example, the skin scar formed after processes of treating trauma, empyrosis, surgeries or plastic surgeries and the like, or cheloid, scleroderma and the like formed in skin of those with scar diathesis. The method is also suitable for repairing of tissue or organ damage and/or wound healing.

In another embodiment, the treating method is a method for treating liver, heart, or renal fibrosis. The method comprises, but not limited to, administering the antibody of the invention or the variant thereof or a drug thereof via a route such as local injection, coating, infiltration or the like

The time of administering of the antibody or variant thereof or the drug of the invention is not particularly limited, preferably immediately after fibrosis occurs. The antibody or the variant thereof or the drug of the invention can also be jointly administered by other systemic or topical application, and the order of administration is not particularly limited.

### Human Pearl Transgenic Animal Model and Drug Screening Method

The invention further provides a novel model for screening and identifying fibrosis drugs. The inventors replaced Pearl gene of a mouse with human Pearl gene through homologous recombination based on the finding of the correlation between Pearl gene and the protein thereof and the fibrotic disease, thereby preparing the model. It should be understood that, according to the teachings of the present invention, a person skilled in the art can also construct other transgenic animals which are replaced by the human Pearl gene in other different manners, and use the transgenic animal as a model for study on fibrosis disease and screening fibrosis drugs.

In addition, the invention further provides a drug screening method. For example, a method for screening Pearl agonist. The method can explores the effect of the Pearl agonist candidate in the fibroblast culture system by using Pearl as a target and the effects thereof in fibroblasts. When the correlation among the activation of Pearl, the epithelial-mesenchymal transition mechanism of the fibroblasts and the extracellular matrix released by fibroblasts is clear, the agonist of the Pearl can be screened out through the drug screening method. Of course, a method for performing pharmacodynamics evaluation by using the transgenic animal model of the present invention is also included, that is, adding a Pearl agonist candidate to the Pearl transgenic animal model of the invention, setting a control, and confirming a potential Pearl agonist while the fibrosis symptoms of the animal are improved. Fibrosis symptoms include, but are not limited to, structural and functional improvements that occur in parts or organs such as lung, heart, kidney, bone marrow, skin, soft tissue, etc.. Said structural and functional improvements can be determined by those skilled in the art (e.g., experimental personnel or clinicians) considering the situation of various parts or organs.

The present invention is further described in detail with the following specific embodiments and the figures. The protection scopes of the present invention are not limited to the following embodiments. Variations and advantages expected by the person skilled in the art could be included in the invention without departing from the spirit and scope of the invention, and the protection scope is defined in the appended claims. Typically, conventional conditions such as Sambrook et al. Molecular Cloning: A Laboratory Manual (New York: Cold Spring Harbor Laboratory Press, 2002), or the conditions suggested by the manufacturer are performed.

### Experimental materials and methods

### 1. Reagent, antibody preparation and cell line

The anti-mouse Pearl and the anti-human Pearl monoclonal antibody were prepared in lab by immunizing knock-out mice and BALB/c mice with human extracellular domain respectively. High-affinity monoclonal hybridoma cells were prepared through standard mouse spleen cells and mouse myeloma cell fusion technology, and then hybridoma cells were injected to the immunodeficient mice to obtain ascites. The ascites were purified through the protein A/G beads and the antibody obtained was used for related experiments. The FcεR1α protein with his tag was purchased from R&D Corporation. The polypeptide is synthesized by GL Biochem (Shanghai) Co. Ltd. Human lung fibroblasts, human skin fibroblasts, cardiac fibroblasts, renal fibroblasts and hepatic fibroblasts are purchased from the ATCC cell library.

### 2. Mouse lung function measurement

Mice are anesthetized by intraperitoneal injection of pentobarbital (100 mg/kg), and each mouse is connected with a computer controlled small animal breathing machine through an air pipe sleeve according to the description of the AniRes2005 lung function analysis system (BestLab version 2.0, AniRes2005, China) manufacturer. The change in pressure in the plethysmography chamber is measured by a port in a connecting tube with a pressure sensor, with a forced vital capacity (FVC) and a 0.1 second forced expiratory volume (FEV 0.1) as an indicator of the lung function.

### 3. Separation and culture of primary mouse lung fibroblasts

The method comprises the following steps: Fresh lung tissues of bleomycin-induced and non-induced mice were shredded, and were then enzymolyzed for 2 hours in a DMEM/F12 culture medium (containing 100 U/mL type I collagenase, 2.5 mg/mL IV collagenase, 0.3 mg/mL hyaluronidase and 0.1 mg/mL DNase). After addition of 2% bovine serum albumin, the suspension of digestion was centrifuged at 800 rpm. Then, the precipitation was suspended with 10% fetal bovine serum DMEM/F12 culture medium, and culturing in a cell culture dish at 37°C with 5% CO₂ for 2 days. The mouse primary lung fibroblasts used in the experiments are cells at the 2-3 generations.

### 4. Plasmid construction and protein expression

The human or mouse Pear1-pcDNA3.1 overexpression plasmids with the flag and his tag were constructed, the plasmids were transfected into 293S cells with PEI, and the 293S cells were cultured in serum-free suspension. The supernatant was extracted, and the protein is purified through the nickel column.

### 5. Flow cytometry

Flow cytometry analysis experiment used fresh lung tissue. Preparing mouse lung single cells by chopping and digesting fresh mouse lung, and collecting the mouse lung single cell by means of a cell filter and centrifuge. The proportion of each cell population was analyzed by incubation with CD45, CD31, CD326, CD140a and Pearl fluorescent labeled antibody. All flow cytometry experiments are carried out by Aria III of a common technical platform in the Shanghai Jiao Tong University, and flow cytometry analysis was carried out using FlowJo software

### 6. Hematoxylin and Eosin (HE) staining and Masson staining

Mouse lung and liver were fixed in 4% paraformaldehyde for 48 hours, after paraffin embedding, a semi-automatic rotary slicing machine (Leica; rm2235 and cm1950) was used for slicing, dewaxing is carried out at room temperature, gradient ethanol is used for rehydration, HE and Masson staining were then performed.

### 7. RNA-seq and Bioinformatics analysis

Total mRNA is extracted from mouse lung CD140⁺ fibroblasts by using Trizol method (Invitrogen, Carlsbad, Calif.) according to standard experimental methods. RNA-SEQ and bioinformatics analysis, differential expression gene (DEG) identification, signaling pathway analysis, gene set enrichment analysis (GSEA) were performed by a common technical service platform of Shanghai Jiao Tong University School of Medicine.

### 8. Quantitative Real-time PCR

Total mRNA was extracted from fibroblasts in accordance with standard protocols using Trizol method (Invitrogen, Carlsbad, Calif.). Complementary DNA is synthesized by reverse transcriptase (Takara). The gene expression is detected by an ABI 7000 Real-Time PCR instrument (Life Technologies). The PCR primer sequences were listed in Table 1. Human glyceraldehyde-3-phosphate dehydrogenase (Gapdh) or 18 sRNA gene was used as control.

**Table 1 qPCR primer sequence**

| Gene | Sense primer | Anti-sense primer |
|---|---|---|
| Mouse Postn | SEQ ID NO: 55 | SEQ ID NO: 56 |
| Mouse Col 4a1 | SEQ ID NO: 57 | SEQ ID NO: 58 |
| Mouse Col 5a3 | SEQ ID NO: 59 | SEQ ID NO: 60 |
| Mouse S100a4 | SEQ ID NO: 61 | SEQ ID NO: 62 |
| Mouse Col 5a2 | SEQ ID NO: 63 | SEQ ID NO: 64 |
| Mouse Des | SEQ ID NO: 65 | SEQ ID NO: 66 |
| Mouse Acta2 | SEQ ID NO: 67 | SEQ ID NO: 68 |
| Mouse Col 1a2 | SEQ ID NO: 69 | SEQ ID NO: 70 |
| Mouse Col 7a1 | SEQ ID NO: 71 | SEQ ID NO: 72 |
| Mouse Loxl2 | SEQ ID NO: 73 | SEQ ID NO: 74 |
| Mouse FN1 | SEQ ID NO: 75 | SEQ ID NO: 76 |
| Mouse Col 24a1 | SEQ ID NO: 77 | SEQ ID NO: 78 |
| Mouse Col 28a1 | SEQ ID NO: 79 | SEQ ID NO: 80 |
| Mouse 18sRNA | SEQ ID NO: 81 | SEQ ID NO: 82 |
| Timp1 | SEQ ID NO: 83 | SEQ ID NO: 84 |
| Human Postn | SEQ ID NO: 85 | SEQ ID NO: 86 |
| Human Col 4a1 | SEQ ID NO: 87 | SEQ ID NO: 88 |
| Human Col 8a1 | SEQ ID NO: 89 | SEQ ID NO: 90 |
| Human Col 3a1 | SEQ ID NO: 91 | SEQ ID NO: 92 |
| Human Col 1a1 | SEQ ID NO: 93 | SEQ ID NO: 94 |
| Human Col 5a2 | SEQ ID NO: 95 | SEQ ID NO: 96 |
| Human Col 5a3 | SEQ ID NO: 97 | SEQ ID NO: 98 |
| Human Col 7a1 | SEQ ID NO: 99 | SEQ ID NO: 100 |
| Human Des | SEQ ID NO: 101 | SEQ ID NO: 102 |
| Human FN1 | SEQ ID NO: 103 | SEQ ID NO: 104 |
| Human 18sRNA | SEQ ID NO: 105 | SEQ ID NO: 106 |

### 9. Statistical method

All data are subjected to statistical analysis by SPSS 13.0 for Windows software. A two-tailed T test is adopted between the two sets of data. Kaplan-Meier analysis was used for survival rate analysis. In all data comparisons, the P value less than 0.05 was considered to have statistical significance.

### Example 1 Construction of Pearl gene knock-out mice

The Pearl gene knock-out mouse (Pearl gene deletion mouse) is constructed through a First-Knockout technology. The construction strategy is shown in FIG. 1a in which an IRES fragment (containing reporter gene lacZ and one promoter driven neo) is inserted into the intron of the original gene, thereby destroying the function of Pearl gene. Mouse genotypes are identified using well-recognized PCR methods in the art. As shown in the sequence of Table 2, the upstream primer (Pearl-F) is designed in the exon 6 region of Pearl gene, and the downstream primer is designed in the insertion fragment Frt region ( Pearl KO-R) or the intron 6 region ( Pearl WT-R) for amplifying the KO strip or the WT strip, respectively. In this invention, 8-10 weeks old mice were used, and mice were fed under specific pathogen-free (SPF) conditions. Mice are approved and used by the Ethics Committee of the Shanghai Jiao Tong University School of Medcine. The construction diagram is shown in FIG. 1a. Western blot data confirmed that the protein in Pearl gene knock-out mouse was completely deleted (FIG. 1b).

**Table 2 mouse genotype identification primers**

| Product | Primer sequence |
|---|---|
| Pear1-F | SEQ ID NO: 107 |
| Pear1-KO-R | SEQ ID NO: 108 |
| Pear1-WT-R | SEQ ID NO: 109 |
| HPear1-WT-F | SEQ ID NO: 110 |
| HPear1-KI-LD-R | SEQ ID NO: 111 |
| HPear1-KI-RD-F | SEQ ID NO: 112 |
| HPear1-WT-R | SEQ ID NO: 113 |

The Pear1-F, Pearl-KO-R and Pear1-WT-R are used for genotype identification of Pearl knock-out mice. HPEAR-1-WT-F, H Pearl-KI-LD-R, H Pear1-KI-RD-F, and H Pear1-WT-R are used for genotype identification of humanized Pearl mice.

### Example 2 Effect of Pearl Deletion on Lung Function of Mice

The lung function of the Pearl gene knock-out mouse in Example 1 is determined. First, the invention compares wild type (WT) and Pearl gene knockout aged mouse lung function and collagen deposition.

The results show that the loss of Pearl obviously caused the decline of the force vital capacity (FVC) and the amount of forced expiratory volume (FEV 0.1) of the 0.1 second in 12 months-old mice (see FIG. 1c). FVC and FEV are important indicators of lung function. The mean value of FVC and FEV0.1 of 12 months-old wild type (WT) control mice is 0.56 and 0.78, and the average value of FVC and FEV 0.1 of 12 months-old Pearl gene knock-out mice is 0.79 and 0.69 (p < 0.1), and therefore, the respiratory function of the aged Pearl gene knockout mouse is significantly worse than that of the WT control mouse.

The immunohistochemical experiment proves that the Pearl gene knockout mouse has more collagen components (see FIG. 1D). Masson staining is a classical method for collagen dyeing, and collagen can be dyed into green; therefore, the deposition condition of collagen can be reflected by the area percentage of green areas; the collagen deposition ratio of 12 months-old WT mice of is 3%, and the collagen deposition proportion of the Pearl gene knock-out mouse is 7%, which obviously exceeded that in wild-type (WT) control mouse.

The results show that the Pearl-deletion old mice can undergo spontaneous lung tissue fibrosis, and the respiratory function of the mouse is damaged.

Example 3: Construction of mouse pulmonary fibrosis model and detection of the influence of Pear 1 deletion on mouse fibrosis.

### 3. Method for constructing mouse lung fibrosis model

Bleomycin (Bleomycin) is a multi-component composite antibiotic with anti-tumor effect, and one of the toxic and side effects is to cause pulmonary fibrosis. Bleomycin is administered in the mouse trachea to induce mouse pulmonary fibrosis, and because pathological histological changes are closest to human lung fibrosis, the mouse model is widely recognized for inducing the pulmonary fibrosis.

In this experiment, the mouse lung fibrosis model is constructed by giving 2 µg/g bleomycin via tracheal spray to WT male mice (8-12 weeks old) and the Pearlknock-out mice in Example 1 to induced lung injury respectively.

### 3. 2. Determination of the influence of Pearl deletion on fibrosis of mice.

It is determined that the survival rate of Pearl gene knockout mouse was much higher than that of wild-type (WT) control mouse during 21 days observation period. The survival rate of the wild-type (WT) control mouse is 0.6 while the survival rate of the wild-type (WT) control mouse is 0.6 after 15 days of induction of bleomycin, while the survival rate of the Pearl gene knockout mouse is only 0.2.

The deletion of Pearl promote bleomycin-induced mouse body weight loss (see FIG. 2b). The body weight of mice was weighed using an electronic scale on day 7, day 14 and day 21 after bleomycin induction. Wherein the weight of the wild-type mouse is reduced from the original 26 g (day 0) to 23.5 g (day 7), 22.5 g (day 14), 21.5 g (day 21), while the weight of the Pearl gene knockout mouse is reduced from 26 g (day 0) to 22 g (day 7), 20.5 g (day 14), 17.5 g (day 21).

The deletion of Pearl significantly aggravated the decline of forced vital capacity and forced expiratory volume in the 0.1 second of the mouse induced by bleomycin (see FIG. 2c). Average values of FVC and FEV0.1 in survival wild-type (WT) control mice were 0.56 and 0.49, while the average values of FVC and FEV 0.1 in Pearl gene knock-out mice were 0.47 and 0.41 (P < 0.01), respectively. So that the respiratory function of the gene knockout mouse is significantly worse than that of the wild-type control mouse.

The immunohistochemical experiment proves that Pearl gene knockout mouse has more collagen components (see FIG. 2d). Masson staining is a classical method for collagen dyeing, and collagen can be dyed into green; therefore, the deposition condition of collagen can be reflected through the area of the green area; and the collagen deposition ratio of wild-type mice on the 21th day after bleomycin induction is 25%, and the collagen deposition proportion of Pearl gene knockout mouse is 48%, which far exceeds the wild-type (WT) control mouse.

According to the experimental result, the deletion ofPear1 can greatly promote the pulmonary fibrosis of the mouse induced by bleomycin, the lung function is remarkably reduced, the collagen deposition is greatly increased, and the death rate is obviously increased.

Example 4 Pearl regulates fibroblast activation and extracellular matrix synthesis by epithelial mesenchymal transition process

After 21 days' bleomycin induction, the wild-type (WT) control mouse and Pearl gene knock-out mouse were subjected to PBS perfusion flushing, the lung was taken down, and then was digested into a single cell suspension with collagenase. By flow cytometry detection of the expression of leukocyte marker CD45, vascular endothelial marker CD31, epithelial marker CD326 and fibroblast marker CD140a, we found that the proportion of the fibroblast of the wild type (WT) control mouse on the 21th day after bleomycin induction is detected to be 16%, and the proportion of the fibroblasts knock-out mouse of Pearl gene is 22%, indicating that the Pearl deletion can obviously promote the increase of the number of fibroblast cells induced by bleomycin (see FIG. 3a).

Next, by the method for culturing the lung single cell suspension on the wall, primary pulmonary fibroblast cells are obtained. Flow cytometry results showed that Pearl was highly expressed in wild-type (WT) control mouse lung fibroblast (see FIG. 3b); the western immunoblotting experiment also verifies the result (see FIG. 3c).

Further, the gene expression of bleomycin-induced and non-induced wild-type (WT) control mice and Pearl gene knock-out mouse lung fibroblast cells was studied by using an RNA-seq technology, a difference gene is subsequently subjected to GSEA enrichment analysis, and the results show that in the case of bleomycin induction, Pearl deletion can greatly promote Epithelial Mesenchymal Transition (EMT) of fibroblasts (see FIG. 3d).

Next, a real-time quantitative PCR technology is used for detecting extracellular matrix protein synthesis genes such as collagen, fibronectin, Periostin (Postn) and so on, and the results showed that in the case of bleomycin induction, the deletion of Pearl greatly promoted the extracellular matrix synthesis capability of fibroblasts.

Therefore, the results of the present example showed that Pearl was an important negative regulatory receptor which naturally inhibits the EMT conversion of fibroblasts and the synthesis of extracellular matrix proteins, and the deletion of Pearl greatly promoted the EMT conversion of fibroblasts and the synthesis capacity of extracellular matrix.

Example 5 Study of Regulation of Lung Tissue-Derived Fibroblast Activation Signaling pathway by Pearl

Preparation of Pearl deletion and non-deleted mouse lung fibroblasts, and then fibroblasts were treated with TGFβ, PDGF and FGF respectively, and key signal molecule phosphorylation (activation) conditions in the fibroblast activation signaling pathway were detected (well-known major signaling pathways of activated fibroblasts comprises a TGFβ receptor-mediated Smad signaling pathway, a MAPK signaling pathway (including ERK1/2, JNK, P38 signaling pathways); a PDGF receptor-mediated MAPK signaling pathway (including ERK1/2, JNK, P38 signaling pathways) and a PI3K/AKT signaling pathway; an FGF receptor-mediated MAPK signaling pathway (including ERK1/2, JNK(P38 signaling pathway) and PI3K/Akt signaling pathway, etc.), it was found that the Perar1 deletion could significantly promote the phosphorylation level of the signal molecules, that is, the intracellular signaling mediated by the TGFβ receptor, the PDGF receptor and the FGF receptor (FIG. 4). FIG. 4 showed that in these signaling pathways, the strip color of the Pearl knock-out mouse (KO) is deeper than that of the wild-type mouse (WT) in which Pearl was normally expressed. So it can be seen that after the Pearl is knocked out, the signaling pathway is over-activated.

The results of the example showed that Pearl can inhibit activation of fibroblasts mediated by various cytokine receptors through intracellular signals.

### Example 6 Activation of inhibits fibroblasts to express extracellular matrix proteins

The above research results showed that Pearl receptor has the ability to inhibit fibroblast proliferation and synthesis of extracellular matrix, and therefore, the inventors believe that Pearl ligand can be used for pulmonary fibrosis treatment under the action on .

In order to investigate whether Pearl ligand reported in the literature includes FcεR1 α , dextran sulfate, and natural fucoidans can inhibit the ability of fibroblasts to synthesize extracellular matrix, the present invention treated human lung fibroblasts with FcεR1α, dextran sulfate and natural fucoidans at the concentrations reported in the literature: 1 µg/ml or 5 µ g/ml of FcεR1α, 5nM or 20nM of dextran sulfate, 30 µg/ml or 300 µg/ml of natural fucoidans.

The results showed that FcεR1α, dextran sulfate and natural fucoidans can inhibit the expression of human lung fibroblast extracellular matrix gene such as bone membrane protein (Periostin), Col5a3 and Timp1 (see FIG. 5). Therefore, the results of the example indicated that activating Pearl could reduce the ability of synthesizing extracellular matrix in fibroblasts.

### Example 7 Construction of Pearl humanized transgenic mice

The human Pearl gene knock-in mouse was constructed by CRISPR/Cas9 technology, a target point was designed near the exon 1-ATG of the mouse coding region, and in the presence of the homologous recombination template, the exogenous gene human was integrated into the target position, and the mouse Pearl gene sequence was replaced. The mouse Pearl has a plurality of transcripts. Therefore, the mouse Pearl gene exon 4-6 was selected to be replaced by the human Pearl cDNA, and an SV40 polyA transcription termination signal is added behind the human Pearl cDNA, so that the mouse Pearl was completely replaced by the human Pearl (see FIG. 7a).

The constructed Pearl humanized transgenic mouse genotype is identified by a recognized PCR method in the art. Lung fibroblasts were collected, a mouse Pearl and a human Pearl level were detected through an immunoblotting method, and the result showed that human Pearl completely replaced the mouse in a humanized transgenic mouse (see FIG. 7b).

In the invention, 8-10 weeks old mice were used, and mice were fed under specific pathogen-free (SPF) conditions. The animal ethics committee of Shanghai Jiao Tong University School of Medcine approves the use of the mice.

### Example 8 Preparation and functional identification of anti-human Pearl antibody

### 8.1 Preparation of anti-human Pearl antibody

Compared with a protein and a compound, the monoclonal antibody has the advantages of strong targeting property, small toxic and side effects, good stability, long in vivo half-life and the like, and has become a hot field of current drug research and development. The invention provides an anti-fibrosis effect of activating fibroblast Pearl through a monoclonal antibody

The method comprises the following steps: firstly, constructing a plasmid and then transfecting suspension cultured 293 cells to express a 6 × His tag human Pearl extracellular segment (Leu-21-Ser; 754); the protein was purified by a nickel column and mixed with an immune adjuvant to immunize Balb/c mice. Separating mouse spleen through an immune standard process; and preparing monoclonal hybridoma cells through standard monoclonal antibody preparation technology.

The invention uses Enzyme-linked immunosorbent assay (ELISA) to screen monoclonal antibody hybridoma cells capable of binding to human Pearl. The monoclonal hybridoma cell strain capable of secreting high-affinity antibody which could bind human Pearl has 53 strains

Identification of the influence of the antibody on extracellular matrix. The effects of the antibody on the expression level of extracellular matrix protein Periotin (Postn), Collarva (Col.4 a1), Col8a1, Col3a1, Col1a1, Col5a2, Col5a3, Col7a1, Desmin (Des) and Actin alpha 2 (Acta2) in human Lung fibroblast (NhLF) were detected using a qPCR method (see table 1 primers).

The six antibodies LF2, LF3, LF7, LF11, LF15 and LF16 could significantly inhibit human lung fibroblasts to express extracellular matrix proteins (see table 3.1).

**Table 3.1 shows the inhibitory effect of the antibody on extracellular matrix ("+" - "+ + +" represents the inhibitory activity from weak to strong, wherein "+" is 20% or less, "+ +" is 20% -50% inhibition intensity, "+ + +" is 50% or more, and Ctrl is an IgG control).**

| Antibody | Periostin (Postn) | Collagen (Col4a1) | Col8a1 | Col3a1 | Col1a1 | Col5a2 | Col5a3 | Col7a1 | Desmin (Des) | Actin alpha 2 (Acta2) |
|---|---|---|---|---|---|---|---|---|---|---|
| LF2 | +++ | ++ | ++ | +++ | +++ | +++ | +++ | +++ | +++ | +++ |
| LF3 | +++ | ++ | +++ | +++ | +++ | ++ | ++ | ++ | ++ | +++ |
| LF7 | +++ | +++ | ++ | +++ | +++ | ++ | ++ | ++ | +++ | +++ |
| LF11 | +++ | ++ | ++ | +++ | +++ | +++ | +++ | +++ | +++ | +++ |
| LF15 | +++ | +++ | +++ | +++ | + | +++ | +++ | +++ | +++ | ++ |
| LF16 | +++ | +++ | +++ | ++ | +++ | +++ | +++ | +++ | +++ | ++ |
| Ctrl | - | - | - | - | - | - | - | - | - | - |

The results show that the mouse monoclonal antibody targeting human can inhibit fibroblasts to synthesize extracellular matrix, and the inhibitory effect of the antibody in the invention disappears under deletion of Pearl gene.

Table 3.2 lists the light chain variable region and heavy chain variable region sequence of the LF2, LF3, LF7, LF11, LF15, LF16 antibody, and the CDR sequences thereof are shown in table 3.3.

**Table3.2**

| Antibody | VH sequence | VL sequence |
|---|---|---|
| LF2 | SEQ ID NO: 1 | SEQ ID NO: 16 |
| LF3 | SEQ ID NO: 2 | SEQ ID NO: 16 |
| LF7 | SEQ ID NO: 3 | SEQ ID NO: 17 |
| LF11 | SEQ ID NO: 2 | SEQ ID NO: 18 |
| LF15 | SEQ ID NO: 4 | SEQ ID NO: 19 |
| LF16 | SEQ ID NO: 5 | SEQ ID NO: 20 |

**Table 3.3**

| Antibody | SEQ ID NO (HCDR1) | SEQ ID NO (HCDR2) | SEQ ID NO (HCDR3) |
|---|---|---|---|
| LF2 | 26 | 27 | 28 |
| LF3 | 26 | 27 | 29 |
| LF7 | 26 | 30 | 28 |
| LF11 | 26 | 27 | 29 |
| LF15 | 31 | 27 | 32 |
| LF16 | 33 | 34 | 35 |

| Antibody | SEQ ID NO (LCDR1) | SEQ (LCDR2) | SEQ ID NO (LCDR3) |
|---|---|---|---|
| LF2 | 45 | GAT | 46 |
| LF3 | 45 | GAT | 46 |
| LF7 | 47 | SAS | 48 |
| LF11 | 49 | DTS | 50 |
| LF15 | 51 | GAT | 52 |
| LF16 | 45 | GAT | 46 |

### 8. 2. Function Identification of Anti-Human Pearl Antibody

8. 2.1 Comparison of antigen binding ability of these antibodies. The ELISA kit is coated with human Pearl ECD protein and then reacts with the anti-Pearl antibody at different concentrations (2000 ng/mL, 1000 ng/mL, 500 ng/mL, 250 ng/mL, 125 ng/mL, 62.5 ng/mL, 31.25 ng/mL, 15.625 ng/mL, 7.8125 ng/mL, 3.90625 ng/mL, 1.953125 ng/mL, 0.9765625 ng/mL) to obtain the EC50 value of antigen-antibody combination (see FIG. 6 and table 3.4 below).

**TABLE 3.4. EC50 Value of Antibody Binding Pearl Extracellular Segment**

| Antiboy | LF2 | LF 3 | LF 7 | LF 11 | LF 15 | LF 16 |
|---|---|---|---|---|---|---|
| EC₅₀ (ng/mL) | 11.11 | 9.269 | 33.65 | 8.908 | 80.73 | 54.73 |

The results show that the binding EC50 of the anti-human Pearl antibody to the is under 100 ng/mL, has a good target binding effect, and even the EC50 of the various antibodies reaches 15 ng/mL or less, and it can be seen that the target specificity of the antibody in the invention is very strong.

### 8. 2.2 The anti-human Pearl antibody has anti-lung fibrosis effects, and improve the lung function.

In order to detect the effect of anti-human Pearl antibody to treat pulmonary fibrosis, the present invention further constructs a transgenic mouse in which human Pearl replaces mouse Pearl, that is, Pearl humanized transgenic mouse, and a construction process refers to example 7 (FIG. 7a). The monoclonal antibody LF2, LF3, LF7, LF11, LF15 and LF16 were suspended in normal saline, and the atomizer was used for atomizing a 1 µg/g antibody to give a bleomycin-induced Pearl humanized transgenic mouse lung for every 7 days (see Example 3). The mice were subjected to lung function and pathological change analysis on the 21th day. Research results show that LF2, LF3, LF7, LF11, LF15 and LF16 are all capable of effectively improving the lung function of the mouse (forced vital capacity, FVC), and the data of which the treatment of the antibody improves the forced vital capacity are shown in table 3.5. Forced vital capacity and lung tissue collagen deposition percentage data of the antibody LF2 treatment group are shown in FIG. 7c and FIG. 7d.

**Table 3.5 Antibody improves the forced vital capacity (FVC) in bleomycin-induced Pearl humanized transgenic mice**

| Antibody(1 µg/g) | Forced vital capacity FVC (Mean, N > 3) | FVC increase (%) |
|---|---|---|
| Control | 0.668 | |
| LF -2 | 0.895 | 25 |
| LF -3 | 0.85 | 21 |
| LF -7 | 0.88 | 24 |
| LF -11 | 0.908 | 26 |
| LF -15 | 0.969 | 31 |
| LF -16 | 0.957 | 30 |

The experimental results show that the specificity of the monoclonal antibody targeting the human Pearl is strong, and the method can be used for relieving the lung fibrosis of the mice induced by bleomycin and improving the lung function.

Example 9 Humanized Transformation and Function Identification of Anti-Human Pearl Antibody

### 9. Construction of humanized antibody

Humanized transformation of the antibody above was carried out according to a CDR transplantation method in the invention, and different back mutations (humanized antibody hLF 101-116, hLF-N55 S, hLF-N55 D, hLF-N55 Q, hLF-G56 A and hLF-G56 V sequences are also shown in Table 4) in the framework region were performed.

**Table 4 Antibody Sequence**

| Antibody | VH Sequence | VL Sequence |
|---|---|---|
| hLF101 | SEQ ID NO: 7 | SEQ ID NO: 22 |
| hLF102 | SEQ ID NO: 7 | SEQ ID NO: 23 |
| hLF103 | SEQ ID NO: 7 | SEQ ID NO: 24 |
| hLF104 | SEQ ID NO: 7 | SEQ ID NO: 25 |
| hLF105 | SEQ ID NO: 8 | SEQ ID NO: 22 |
| hLF106 | SEQ ID NO: 8 | SEQ ID NO: 23 |
| hLF107 | SEQ ID NO: 8 | SEQ ID NO: 24 |
| hLF108 | SEQ ID NO: 8 | SEQ ID NO: 25 |
| hLF109 | SEQ ID NO: 9 | SEQ ID NO: 22 |
| hLF110 | SEQ ID NO: 9 | SEQ ID NO: 23 |
| hLF111 | SEQ ID NO: 9 | SEQ ID NO: 24 |
| hLF112 | SEQ ID NO: 9 | SEQ ID NO: 25 |
| hLF113 | SEQ ID NO: 10 | SEQ ID NO: 22 |
| hLF114 | SEQ ID NO: 10 | SEQ ID NO: 23 |
| hLF115 | SEQ ID NO: 10 | SEQ ID NO: 24 |
| hLF116 | SEQ ID NO: 10 | SEQ ID NO: 25 |
| hLF-N55S | SEQ ID NO: 11 | SEQ ID NO: 22 |
| hLN55D | SEQ ID NO: 12 | SEQ ID NO: 22 |
| hLF-N55Q | SEQ ID NO: 13 | SEQ ID NO: 22 |
| hLF-G56A | SEQ ID NO: 14 | SEQ ID NO: 22 |
| hLF-G56V | SEQ ID NO: 15 | SEQ ID NO: 22 |

The CDR sequence of the antibody is shown in SEQ ID NO: 26-55 in Table 5, and the CDR sequence of the antibody adopts an IMGT numbering rule.

**Table 5 CDR sequence under antibody IMGT numbering rule**

| Antibody name | SEQ ID NO (HCDR1) | SEQ ID NO (HCDR2) | SEQ ID NO (HCDR3) |
|---|---|---|---|
| LF2 | 26 | 27 | 28 |
| hLF 101-106 | 39 | 27 | 28 |
| hLF-N 55 S | 39 | 40 | 28 |
| hLF-N 55 D | 39 | 41 | 28 |
| hLF-N 55 Q | 39 | 42 | 28 |
| hLF-G56 A | 39 | 43 | 28 |
| hLF-G56 V | 39 | 44 | 28 |

| Antibody name | SEQ ID NO (LCDR1) | SEQ ID NO (LCDR2) | SEQ ID NO (LCDR3) |
|---|---|---|---|
| LF2 | 45 | GAT | 46 |
| hLF101-116 | 45 | GAT | 46 |
| hLF-N55S | 45 | GAT | 46 |
| hLF-N55D | 45 | GAT | 46 |
| hLF-N55Q | 45 | GAT | 46 |
| hLF-G56A | 45 | GAT | 46 |
| hLF-G56V | 45 | GAT | 46 |

### ]9.2 The binding affinity test of the humanized anti- antibody to the extracellular segment (see Table 6).

**Table 6: Biacore data of humanized antibodies in combination with extracellular segments**

| Ligand | ka (1/Ms) | kd (1/S) | t1/2 (s) | KD (M) | Rmax (RU) |
|---|---|---|---|---|---|
| hLF 101 | 5.12E+04 | 3.93E-04 | 1765.3 | 7.67E-09 | 13.8 |
| hLF 102 | 5.02E+04 | 3.95E-04 | 1753.4 | 7.87E-09 | 17.5 |
| hLF 103 | 2.80E+04 | 5.32E-04 | 1303.5 | 1.90E-08 | 15 |
| hLF 104 | 3.80E+04 | 5.06E-04 | 1369.8 | 1.33E-08 | 22.2 |
| hLF 105 | 4.62E+04 | 1.76E-04 | 3934.1 | 3.82E-09 | 22.2 |
| hLF 106 | 4.01E+04 | 2.33E-04 | 2980.3 | 5.80E-09 | 14.6 |
| hLF 107 | 5.12E+04 | 3.45E-04 | 2008.2 | 6.74E-09 | 24.6 |
| hLF 108 | 5.13E+04 | 3.07E-04 | 2260.8 | 5.98E-09 | 18.7 |
| hLF 109 | 4.05E+04 | 2.49E-04 | 2786.8 | 6.14E-09 | 18.7 |
| hLF 110 | 4.35E+04 | 2.36E-04 | 2942.4 | 5.42E-09 | 25.4 |
| hLF 111 | 3. 95E+04 | 2.71E-04 | 2556.8 | 6.87E-09 | 15.4 |
| hLF 112 | 4.75E+04 | 2.37E-04 | 2923.1 | 4.99E-09 | 20.4 |
| hLF 113 | 4.70E+04 | 2.01E-04 | 3446.2 | 4.28E-09 | 24.7 |
| hLF 114 | 4.56E+04 | 2.32E-04 | 2986.5 | 5.09E-09 | 19.5 |
| hLF 115 | 4.42E+04 | 2.35E-04 | 2953.9 | 5.31E-09 | 21 |
| hLF 116 | 4. 34E+04 | 2.21E-04 | 3136.2 | 5.09E-09 | 28.2 |
| LF2-hlgG 4 | 5. 31E+04 | 1. 87E-04 | 3705.9 | 3.52E-09 | 21 |

The results show that each humanized antibody has the binding activity with the extracellular segment of Pearl, and the replacement and back mutation of FR region of the antibody have little effect on the CDR's affinity.

### 9.3 The role of humanized anti- Pearl antibody in bleomycin-induced mouse lung fibrosis

In the invention, the above humanized modified antibody was suspended in normal saline, and the atomizer is used for atomizing the 1 µg/g humanized modified antibody to the bleomycin-induced Pearl humanized transgenic mouse lung (see Example 3). The mice are subjected to lung function analysis on the 21th day. The research results show that the above antibody can effectively improve the lung function of the mouse (the forced vital capacity FVC), and the data is shown in Table 7.

**Table 7 Humanized anti-Pearl antibodies improve the forced vital capacity (FVC) of bleomycin-induced Pearl humanized transgenic mice.**

| Antibody (1 µg/g) | FVC (Mean, N > 3) | FVC increase % |
|---|---|---|
| Control | 0.668 | |
| hLF 101 | 0.899 | 26 |
| hLF 102 | 0.965 | 31 |
| hLF 103 | 0.886 | 25 |
| hLF 104 | 1.045 | 36 |
| hLF 105 | 1.036 | 36 |
| hLF 106 | 1.133 | 41 |
| hLF 107 | 1.003 | 33 |
| hLF 108 | 0.912 | 27 |
| hLF 109 | 1.005 | 34 |
| hLF 110 | 0.989 | 32 |
| hLF 111 | 1.003 | 33 |
| hLF 112 | 1.023 | 35 |
| hLF 113 | 0.897 | 26 |
| hLF 114 | 0.965 | 31 |
| hLF 115 | 1.016 | 34 |
| hLF 116 | 0.935 | 29 |
| hLF-N55S | 1.005 | 34 |
| hLF-N55D | 0.798 | 16 |
| hLF-N55Q | 0.968 | 31 |
| hLF-G56A | 0.997 | 33 |
| hLF-G56V | 0.925 | 28 |

The results show that all humanized antibodies can effectively improve the forced vital capacity of bleomycin-induced Pearl humanized mice.

Example 10 Anti-human Pearl monoclonal antibody inhibits various tissue-derived fibroblasts to express extracellular matrix proteins

In order to further study the effect of Pearl in other source fibroblasts. In the invention, real-time quantitative PCR technology was utilized, and the influences of Pearl monoclonal antibody on the extracellular matrix synthetic capacity in human skin fibroblasts, cardiac fibroblasts, renal fibroblasts and hepatic fibroblasts were studied.

The research results show that the anti-human Pearl antibody in this invention can also inhibit cultured human skin fibroblasts, cardiac fibroblasts, renal fibroblasts and hepatic fibroblasts to express the extracellular matrix. The LF 3 monoclonal antibody inhibits human kidney fibroblasts to express extracellular matrix proteins (see FIG. 8a). The LF 3 monoclonal antibody inhibits human cardiac fibroblasts to express extracellular matrix proteins (see FIG. 8b).The LF 3 monoclonal antibody inhibits human liver fibroblasts to express extracellular matrix proteins (see FIG. 8c). The LF 3 monoclonal antibody inhibits human skin fibroblasts to express extracellular matrix proteins (see FIG. 8d).

The above results further suggest that the antibody acting on fibroblasts Pearl can also be used to treat skin scar formation in the processes of trauma, empyrosis, surgery, or shaping and beauty, treat the cardiac fibrosis, the heart failure diastolic function or ejection fraction reduce and arrhythmia caused by cardiac injury caused by aging or other reasons, treat kidney fibrosis caused by kidney injury, treat liver fibrosis caused by various infectious and chemical damage, and treat liver fibrosis-induced portal vein high-pressure, liver ascites, cirrhosis, gated pulmonary hypertension, treat bone marrow fibrosis caused by transplantation or chemotherapy, treat pancreatic fibrosis, etc. and treat spleen, nervous system fibrosis, etc.

While specific examples of the present invention have been described above, it should be understood by those technical personnel in the art that these are just examples and various changes or modifications may be made to these examples without departing from the principles and spirit of the invention. Therefore, the scope of the invention is defined by the appended claims.

## Claims

1. Application of Pearl gene, encoded protein or agonist thereof in 1) preparing of a drug for treating fibrotic diseases, 2) preparing of a drug for down-regulating and/or inhibiting fibroblast activation signaling pathway, and/or 3) preparing of a drug for inhibiting fibroblast activation.

2. The application of claim 1, wherein the fibrotic disease comprises pulmonary fibrosis, hepatic fibrosis, cardiac fibrosis, renal fibrosis, bone marrow fibrosis, skin scar and/or soft tissue scar.

3. An isolated antibody or a variant thereof targeting Pear 1, the antibody comprises a heavy chain variable region and/or a light chain variable region; amino acid sequences of HCDR1, HCDR2 and HCDR3 of the heavy chain variable region are shown as SEQ ID NO: 26, SEQ ID NO: 27, and SEQ ID NO: 28, respectively; amino acid sequences of LCDR1, LCDR2 and LCDR3 of the light chain variable region are shown as SEQ ID NO: 45, GAT and SEQ ID NO: 46, respectively, or amino acid sequences of LCDR1, LCDR2 and LCDR3 of the light chain variable region are shown as SEQ ID NO: 47 GTS, and SEQ ID NO: 48, respectively, or amino acid sequences of LCDR1, the LCDR2 and the LCDR3 of the light chain variable region are shown as SEQ ID NO: 49, DTS, and SEQ ID NO: 50, respectively, or amino acid sequences of LCDR1, LCDR2 and LCDR3 of the light chain variable region are shown as SEQ ID NO: 51, GAT, and SEQ ID NO: 52, respctively, or amino acid sequences of LCDR1, LCDR2 and LCDR3 of the light chain variable region are shown as SEQ ID NO: 53, LVS, and SEQ ID NO: 54, respcetively.

4. An isolated nucleic acid encoding any one of the antibodies or the variant thereof of claim 3.

5. A recombinant expression vector comprising the isolated nucleic acid of claim 4.

6. A transformant comprising the isolated nucleic acid of claim 4 or the recombinant expression vector of claim 5.

7. A drug comprising the antibody or the variant thereof of claim 3.

8. Application of the antibody or the variant thereof of claim 3, the nucleic acid of claim 4, the recombinant expression vector of claim 5, the transformant of claim 6, and/or the drug of claim 7 in 1) preparing of a drug for treating fibrotic diseases, 2) preparing of a drug for down-regulating and/or inhibiting fibroblast activation signaling pathway, and/or 3) preparing of a drug for inhibiting fibroblast activation.

9. A method for constructing a humanized Pearl transgenic animal model comprising the step of replacing an animal Pearl gene with an human Pearl gene.

10. A method for screening a Pearl agonist comprising the steps of (1) adding or not adding candidate molecules into a fibroblast culture system of a test group or a control group, respectively; (2) comparing fibroblast activation between the test group and the control group; and (3) confirming the candidate molecule is a Pearl agonist if the fibroblast activation in the test group is significantly lower than the control group.
